(19) Europäisches Patentamt

European Patent Office

Office européen des brevets

(11) **EP 0 915 910 B1**

(12) **EUROPÄISCHE PATENTSCHRIFT**

(45) Veröffentlichungstag und Bekanntmachung des Hinweises auf die Patenterteilung:
**18.01.2006 Patentblatt 2006/03**

(21) Anmeldenummer: **97927143.4**

(22) Anmeldetag: **05.06.1997**

(51) Int Cl.:
**C07K 14/575** (2006.01)     **A61K 38/22** (2006.01)

(86) Internationale Anmeldenummer:
**PCT/EP1997/002930**

(87) Internationale Veröffentlichungsnummer:
**WO 1997/046584 (11.12.1997 Gazette 1997/53)**

(54) **EXENDIN-ANALOGA, VERFAHREN ZU DEREN HERSTELLUNG UND DIESE ENTHALTENDE ARZNEIMITTEL**

EXENDIN ANALOGUES, PROCESSES FOR THEIR PREPARATION AND MEDICAMENTS CONTAINING THEM

ANALOGUES EXENDINE, PROCEDES PERMETTANT DE LES PREPARER ET MEDICAMENTS LES CONTENANT

(84) Benannte Vertragsstaaten:
**AT BE CH DE DK ES FI FR GB GR IE IT LI LU NL PT SE**

(30) Priorität: **05.06.1996 DE 19622502**
**13.09.1996 DE 19637230**

(43) Veröffentlichungstag der Anmeldung:
**19.05.1999 Patentblatt 1999/20**

(73) Patentinhaber: **Roche Diagnostics GmbH**
**68305 Mannheim (DE)**

(72) Erfinder:
• **HOFFMANN, Eike**
**D-68519 Viernheim (DE)**
• **GÖKE, Rüdiger**
**D-35043 Marburg (DE)**
• **GÖKE, Burkhard-Johannes**
**D-35637 Marburg (DE)**

(74) Vertreter: **Poppe, Regina**
**F.Hoffmann-La Roche AG**
**Patent Department (CLP)**
**Grenzacherstrasse 124**
**4070 Basel (CH)**

(56) Entgegenhaltungen:
**WO-A1-98/05351          WO-A1-98/30231**
**US-A- 5 424 286**

• **K. ADELHORST ET AL.: "Structure-Activity studies of Glucagon-like Peptide-1 (GLP-1)" JOURNAL OF BIOLOGICAL CHEMISTRY, Bd. 269, Nr. 9, 4.März 1994, MD US, Seiten 6275-6278, XP002045291**
• **J. ENG ET AL: "Purification and structure of Exendin-3" JOURNAL OF BIOLOGICAL CHEMISTRY., Bd. 265, Nr. 33, 25.November 1990, MD US, Seiten 20259-20262, XP002045292**
• **J. ENG ET AL: "Isolation of exendin-4" J. BIOL. CHEM, Bd. 267, Nr. 11, 15.April 1992, Seiten 7402-7405, XP002045293**

**Beschreibung**

Diese Erfindung betrifft neue Exendin-Analoga, welche bei der Therapie des Diabetes mellitus eingesetzt werden können, Verfahren zu deren Herstellung und diese enthaltende Arzneimittel.

**Hintergrund der Erfindung**

Eine funktionelle Verbindung zwischen Dünndarm und exokrinem Pankreas wurde in den sechziger Jahren bewiesen, nachdem die exakte Bestimmung von Insulin im Plasma möglich wurde. Die Insulinantwort nach oraler Glukosegabe ist wesentlich kräftiger als die nach intravenöser Glukoseapplikation, auch wenn identische Glukoseplasmaspiegel erreicht werden. Diesen "Inkretin-Effekt" erklärt man im funktionellen Verbund der entero-insulären Achse. Verantwortlich für diesen Effekt sind Darmhormone, die nach Mahlzeiten vom Dünndarm freigesetzt, erhöht meßbar im Plasma zirkulieren und die Glukose-induzierte Insulinfreisetzung verstärken. Neben dem klassischen Inkretinhormon "Gastric inhibitory polypeptide I" (GIP), ist heute "Glucagon-like peptide-1" (GLP-1) in den Vordergrund des Interesses gerückt. In relativ kurzer Zeit ist GLP-1 vom physiologisch interessantesten Inkretinhormon-Kandidaten zur potentiellen Alternative zur Behandlung des Diabetes mellitus Typ II gereift. Die vorliegende Erfindung beschreibt neue Substanzen, die dem natürlich vorkommenden GLP-1 Molekül in der Wirkung nachempfunden sind. Die neuen Substanzen zeichnen sich durch erhöhte Stabilität bei erhaltener Wirksamkeit aus.

**Antidiabetogene Wirkung**

Infusion und subkutane Injektion von GLP-1 bewirken bei Patienten mit Diabetes mellitus Typ II eine deutliche Steigerung der Insulinsekretion sowie eine Hemmung der Glukagonfreisetzung (Gutniak, M. (1992); Kreymann. B. (1987); Nathan, D.M. (1992); Nauck, M.A. (1993a & b)). Beides ist aus therapeutischer Sicht von Interesse und an der blutzuckersenkenden Wirkung von GLP-1 beteiligt: Insulin fördert an seinen Zielgeweben die Glukoseaufnahme sowie eine Hemmung der Glukoneogenese. Desweiteren ist bei GLP-1-Analogen eine Verstärkung der Glukoseaufnahme in der Peripherie zu erwarten. Die Hemmung der Glukagonsekretion muß als indirekte GLP-1-Wirkung angesehen werden, da Glukagon-produzierende A-Zellen keine GLP-1 Rezeptoren exprimieren (Komatsu, R. (1989)). Vielmehr scheint dafür die erhöhte Insulin- und Somatostatinfreisetzung ausschlaggebend zu sein. Beide Hormone sind als Hemmstoffe der Glukagonfreisetzung bekannt.

Sicherlich tragen zwei molekulare Mechanismen zur GLP-1-induzierten Insulinfreisetzung bei Diabetes mellitus Typ II bei. Neben der direkten Verstärkung der Glukose-induzierten Insulinfreisetzung sensibilisiert GLP-1 eine Subgruppe von B-Zellen gegenüber dem Schlüsselreiz "Glukose" (Fehmann, H.C. (1991)) und möglicherweise auch gegenüber weiteren Stimuli, so daß insgesamt mehr B-Zellen Insulin sezernieren. Diese "Priming"-Wirkung erklärt am ehesten die Tatsache, daß GLP-1 trotz seiner relativ kurzen Plasma-Halbwertszeit zu einer langanhaltenden Insulinfreisetung führt.

Diese Wirkung ist abhängig von erhöhten Glukose-Spiegeln (> 108 mg/dl) (Göke, R. (1993a)). Sie unterscheidet GLP-1 grundsätzlich von den Sulfonylharnstoffen, die die Insulinsekretion unabhängig vom Glukose-Plasmaspiegel beeinflussen: Sinkt der Glukosewert unter 108 mg/dl, so versiegt die Insulinsekretion selbst bei intravenöser Infusion von GLP-1. Daher sind beim therapeutischen Einsatz von GLP-1 kaum Hypoglykämien zu erwarten. Tatsächlich wurden sie in den bisherigen klinischen Studien auch nicht beschrieben. Problematisch sind allerdings die pharmakokinetischen Eigenschaften von GLP-1. Aufgrund seiner sehr kurzen Halbwertszeit ist die Wirkdauer nur begrenzt.

Aus therapeutischer Sicht ist in jedem Fall die Synthese stabiler und wirkungsstarker GLP-1 analoger Peptide wünschenswert. Es wurden nun auf der Basis des ursprünglich aus dem Gift von Echsen isolierten Moleküls Exendin Peptidanaloga synthetisiert, mit dem Ziel verbesserte abbaustabilisierte Therapeutika mit verlängerter Wirkdauer zur behandlung des Diabetes mellitus zu entwickeln. Diese Peptide haben die gleiche pharmakologische Wirkung wie GLP-1, weisen aber überraschenderweise eine deutlich längere Halbwertszeit auf.

Die als Gegenstand der Erfindung beschriebenen neuen Peptidsequenzen zeigen Wirkung auf Insulinsynthese und -abgabe sowie Wirkung auf den Insulineffekt insbesondere die Glucoseaufnahme in den Zielgeweben Muskel- und Fettgewebe, sowie der Magenentleerung.

**Gegenstand der Erfindung**

Die vorliegende Erfindung basiert auf der Sequenz von Exendin-3 und Exendin-4, Peptiden, welche aus dem Sekret von *Heloderma horridum* bzw. *Heloderma suspectum* isoliert wurden (Eng, J. et al. (1990,1992)). Die Aminosäuren-Sequenz und Wirkung der beiden Peptide am Pankreas wurde bereits von mehreren Autoren publiziert (Eng, J. et al. (1990); Raufman, J. P.(1992); Göke, R. (1993b); Thorens, B. (1993)). US Patent Nr. 5, 424,286 (Eng, J.) beschreibt die Exendin-Fragmente Exendin-4(1-31) und Exendin-4(9-39) sowie ein Exendin-4(1-31), in dem die Aminosäure 31 durch Tyrosin ausgetauscht wurde.

Gegenstand dieser Erfindung sind neue verkürzte Exendin-Fragmente, welche die Aminosäuresequenzen von Exendin-3-(1-30), oder Exendin-4-(1-30) umfassen, wobei das C-terminale Ende dieser Sequenzen um bis zu 3 Aminosäuren verkürzt, vorzugsweise um höchstens 1 Aminosäure, und das N-terminale Ende um bis zu 2, vorzugsweise höchstens 1 Aminosäure, verkürzt sein kann. Überraschenderweise sind diese Exendin-Fragmente biologisch wirksam, obwohl die Aminosäuresequenz verkürzt ist. Verkürzte Aminosäuresequenzen sind wirtschaftlicher herzustellen als vergleichsweise längere Sequenzen. Besonders bevorzugt sind also Peptidfragmente mit den folgenden Sequenzen; insbesondere sind die Peptidfragmente, die auf Exendendin-3 (1-30) (Seq. ID No.I) beruhen:

### SEQ ID NO: 1  basiert auf Exendin-3

| 1 | | | | 5 | | | | | 10 | | | | | 15 | | | |
|---|---|---|---|---|---|---|---|---|---|---|---|---|---|---|---|---|---|
| H | S | D | G | T | F | T | S | D | L | S | K | Q | M | E | E | E | A | V |

| 20 | | | | | 25 | | | | 30 |
|---|---|---|---|---|---|---|---|---|---|
| R | L | F | I | E | W | L | K | N | G | $X_1$ |

### SEQ ID NO: 2  basiert auf Exendin-4

| 1 | | | | 5 | | | | | 10 | | | | | 15 | | | |
|---|---|---|---|---|---|---|---|---|---|---|---|---|---|---|---|---|---|
| H | G | E | G | T | F | T | S | D | L | S | K | Q | M | E | E | E | A | V |

| 20 | | | | | 25 | | | | 30 | | | | 35 | | | 38 |
|---|---|---|---|---|---|---|---|---|---|---|---|---|---|---|---|---|
| R | L | F | I | E | W | L | K | N | G | $X_1$ |

wobei die Aminosäuren an Position 1, 2, 28, 29 oder 30 je nach gewünschter Kettenlänge Teil der Sequenz sein können. Die Peptide sind vom N-Terminus zum C-Terminus durchnumeriert. $X_1$ bedeutet eine proteogene oder nichtproteogene Aminosäure außer Glycin.

Bevorzugt sind Exendin- und Exendinanaloga mit einer Kettenlänge von 1-27, besonders bevorzugt solche mit einer Kettenlänge 1-30.

Die Carboxylgruppe $COR_3$ der Aminosäure am C-terminalen Ende kann in freier Form ($R_3$ = OH) oder in Form eines physiologisch verträglichen Alkali- oder Erdalkalisalzes, wie z. B. des Natrium-, Kalium- oder Calciumsalzes vorliegen. Die Carboxylgruppe kann auch mit primären, sekundären oder tertiären Alkoholen verestert sein, wie z. B. Methanol, verzweigten oder unverzweigten $C_1$-$C_6$-Alkylalkoholen, insbesondere Ethylalkohol oder tert.-Butanol. Die Carboxylgruppe kann aber auch mit primären oder sekundären Aminen amidiert sein, wie z. B. Ammoniak, verzweigten oder unverzweigten $C_1$-$C_6$-Alkylaminen oder $C_1$-$C_6$-Di-Alkylaminen, insbesondere Methylamin oder Dimethylamin.

Die Aminogruppe der Aminosäure $NR_1R_2$ am N-terminalen Ende kann in freier Form ($R_1$, $R_2$ = H) oder in Form eines physiologisch verträglichen Salzes, wie z. B. des Chlorides oder Acetats vorliegen. Die Aminogruppe kann auch mit Säuren acetyliert sein, so daß $R_1$ = H und $R_2$ = Acetyl-, Trifluoracetyl-, Adamantyl-, oder durch die gängigen Aminoschutzgruppen der Peptidchemie, wie z. B. Fmoc, Z, Boc, Alloc geschützt vorliegen, oder N-alkyliert sein mit $R_1$ und/oder $R_2$ = $C_1$-$C_6$- Alkyl oder $C_2$-$C_8$-Alkenyl oder $C_7$-$C_9$-Aralkyl.

Unter Alkylresten werden geradkettige, verzweigte oder gegebenenfalls ringförmige Alkylreste verstanden, vorzugsweise Methyl, Ethyl, Isopropyl und Cyclohexyl.

Alle Exendin-Fragmente können als voll oder partiell geschützte Derivate vorliegen.

Gegenstand dieser Erfindung sind außerdem Exendin-Fragmente mit den oben genannten Eigenschaften und Sequenzlängen, bei denen zusätzlich mindestens eine aber maximal elf der unter (a) bis (p) aufgeführten Modifikationen durchgeführt wurden. Bevorzugt sind solche Exendin-Fragmente, die maximal 9, besonders bevorzugt sind solche, die maximal 5 der unter (a) bis (p) aufgeführten Modifikationen aufweisen.

(a) Die α-Aminosäure in Position 1 ist Ala, Gly oder Lys; oder

(b) Die α-Aminosäure in Position 2 ist Ser, Thr, Gly, Ala, Val, Ile oder Leu; oder

(c) Die α-Aminosäure in Position 3 ist Glu, Asp oder Ala; oder

(d) Die Aminosäure in Position 4 ist Ala; oder

(e) Die α-Aminosäure in Position 5 ist Ser, Tyr oder Ala; oder

(f) Die α-Aminosäure in Position 6 ist Ala, Ile, Val, Leu oder Tyr; oder

(g) Die α-Aminosäure in Position 7 ist Ala, Tyr oder Ser; oder

(h) Die α-Aminosäure in Position 8 ist Ala, Tyr oder Thr; oder

(i) Die α-Aminosäure in Position 9 ist Ala oder Glu; oder

(j) Die Aminosäuren in Position 10, 11, 12. 15, 16, 17,18, 19, 20, 21, 24, 28, 29 sind unabhängig voneinander eine proteinogene L-Aminosäure; oder

(k) Die α-Aminosäure in Position 13 ist eine neutrale proteinogene L-Aminosäure; oder

(l) Die α-Aminosäure in Position 14 wird ersetzt durch Nle, D-Nle, Ala, D-Ala. Ile, D-Ile, Val oder D-Val, besonders bevorzugt sind Ile, Val oder Ala; oder

(m) Die α-Aminosäure in Position 22 ist Tyr, Leu oder Val; oder

(n) Die α-Aminusäure in Position 23 ist Leu, Val, Tyr oder Phe; oder

(o) Die α-Aminosäure in Position 25, 26 oder 27 ist eine neutrale, proteinogene L-Aminosäure; oder

(p) Die α-Aminosäure in Position 30 ist Arg oder Tyr.

Unter den neuen Exendin-Fragmenten sind solche besonders bevorzugt, welche neben den schon genannten Eigenschaften und Sequenzlängen, an Position 10 die Aminosäure Leucin und/oder an Position 19 die Aminosäure Valin, an Position 14 anstelle von Methionin die Aminosäure Isoleucin oder Alanin und an Position 30 Arginin enthalten. Besonders bevorzugt sind auch diejenigen Modifikationen von Exendin-Fragmenten, bei denen sich, zusätzlich zu den erwähnten besonders bevorzugten Aminosäuren an den Positionen 10, 14, 19 und 30, an der Position 2 eine der 20 bekannten proteinogenen L-Aminosäuren befindet.

Bevorzugte Exendinanaloga weisen einen Austausch in Position 3 oder 14 auf, besonders bevorzugt in Position 2, ganz besonders bevorzugt enthalten die Exendinanaloga nur proteinogene Aminosäuren.

Gegenstand der Erfindung sind neben neuen verkürzten und stabilisierten Exendin-3 und Exendin-4 Analoga auch Verfahren zur Herstellung dieser Analoga, bei denen man die Analoga in Festphasensynthese aus geschützten, in den Analoga enthaltenen Aminosäuren, herstellt, die in der Reihenfolge aneinander gekoppelt werden, welche den Aminosäuresequenzen in den Analoga entsprechen und welche gegebenenfalls durch entsprechende nicht in den natürlichen Exendin-Peptiden vorkommende Aminosäuren ergänzt wurden.

Das Glycin in Position 30, der Exendin-3 oder Exendin-4-Sequenz wurde gegen eine andere proteogene oder nicht-proteogene Aminosäure ausgetauscht, um bei der Synthese nach der Abspaltung der aminoterminalen Schutzgruppe, keine Diketopiperazinbildung vorliegen zu haben.

Die Exendin-(1-30)-Analoga und Fragmente sind gegenüber den Exendinen-1(1-39) vorteilhaft, da durch die kürzeren Sequenzen diese Analoga einfacher und in höheren Ausbeuten synthetisiert werden können.

Die verwendeten Abkürzungen und Definitionen der Aminosäuren wurden in Pure Appl. Chem. 31, 639-45 (1972) und ibid. 40, 277-90 (1974) empfohlen und stimmen mit den PCT-Regeln (WIPO Standard St. 23: Recommendation for the Presentation of Nucleotide and Amino Acid Sequences in Patent Applications and in Published Patent Documents) überein. Die Ein- bzw. Drei-Buchstabencodes sind wie folgt:

|  | Aminosäureabkürzungen |  |
|---|---|---|
| Aminosäure | Drei-Buchstaben-Code | Ein-Buchstaben-Code |
| Alanin | Ala | A |
| Arginin | Arg | R |
| Asparagin | Asn | N |
| Asparaginsäure | Asp | D |
| Cystein | Cys | C |
| Glutamin | Gln | Q |
| Glutaminsäure | Glu | E |
| Glycin | Gly | G |
| Histidin | His | H |
| Isoleucin | Ile | 1 |
| Leucin | Leu | L |
| Lysin | Lys | K |
| Methionin | Met | M |

Tabelle fortgesetzt

| Aminosäure | Drei-Buchstaben-Code | Ein-Buchstaben-Code |
|---|---|---|
| Phenylalanin | Phe | F |
| Prolin | Pro | P |
| Serin | Ser | S |
| Threonin | Thr | T |
| Tryptophan | Trp | W |
| Tyrosin | Tyr | Y |
| Valin | Val | V |
| andere Aminosäuren | Xaa | X |

Die Abkürzungen stehen für L-Aminosäuren, falls keine weiteren Spezifikationen wie D- oder D,L- angegeben sind. D-Aminosäuren werden im Ein-Buchstabencode mit kleinen Buchstaben geschrieben. Bestimmte Aminosäuren, natürliche wie nichtnatürliche sind achiral, z. B. Glycin. Bei der Darstellung aller Peptide befindet sich das N-terminale Ende links und das C-terminale Ende rechts.

Beispiele für nichtproteinogene Aminosäuren sind in folgender Auflistung mit ihren Abkürzungen angegeben:

| | |
|---|---|
| β-Alanin | β-Ala |
| o-Aminobenzoesäure | Oab |
| m-Aminobenzoesäure | Mab |
| p-Aminobenzoesäure | Pab |
| m-Aminomethylbenzoesäure | Amb |
| ω-Aminohexansäure | Ahx |
| ω-Aminoheptansäure | Ahp |
| ω-Aminooctansäure | Aoc |
| ω-Aminodecansäure | Ade |
| ω-Aminotetradecansäure | Atd |
| Citrutlin | Cit |
| Cyclohexylalanin | Cha |
| $\alpha,\gamma$-Diaminobuttersäure | Dab |
| $\alpha,\beta$-Diaminopropionsäure | Dap |
| Methionin-Sulfoxid | Met(O) |
| $C^{\alpha}$-Methyl-Alanin | Aib |
| N-Methyl-Glycin (Sarkosin) | Sar |
| Naphtylalanin | Nal |
| Norleucin | Nle |
| Omithin | Orn |
| 1,2,3,4-Tetrahydroisochinolin-3-carbonsäure | Tic |

Alle Aminosäuren lassen sich nach ihren physikalisch-chemischen Eigenschaften in die folgenden drei Hauptklassen unterteilen:

Sauer: Die Aminosäure gibt in wässriger Lösung und bei physiologischem pH ein Proton ab und trägt infolgedessen eine negative Ladung.
Basisch: Die Aminosäure nimmt in wässriger Lösung und bei physiologischem pH ein Proton auf und trägt infolgedessen eine positive Ladung.
Neutral: Die Aminosäure ist in wässriger Lösung und bei physiologischem pH in einem ungeladenen Zustand.

Die Definition "trägt eine positive/negative Ladung" oder "ist im ungeladenen Zustand" trifft dabei dann zu, wenn im statistischen Mittel eine signifikante Anzahl von Aminosäuren einer Klasse (mindestens 25%) sich im genannten Zustand befindet

Neben den 20 sogenannten proteinogenen Aminosäuren, deren Einbau in Proteine durch die Information des genetischen Codes geregelt ist, lassen sich auch nicht proteinogene über die beschriebenen Syntheseverfahren in Peptidsequenzen einbauen. Eine Aufzählung der proteinogenen Aminosäuren und deren Einteihmg in die oben genannten drei Klassen ist in Tabelle 1 gegeben. Nicht-proteinogene Aminosäuren sind nicht genetisch codiert. Beispiele für nicht-proteinogene Aminosäuren und deren Einteilung in saure, basische oder neutrale Aminosäuren sind in Tabelle 1 gegeben.

**Tabelle 1**

|  | proteinogen | nicht-proteinogen |
|---|---|---|
| sauer | Asp, Glu | |
| basisch | Arg, His, Lys | Dab, Dap, Orn |
| neutral | Ala, Asn, Cys, Gln, Gly, Ile, Leu, Met, Phe, Pro, Ser, Thr, Trp, Tyr, Val | ß-Ala, Aib, Cit, Cha, Oab, Mab, Pab, Amb, Ahx, Ahp, Aoc, Ade, Acd, Nal, Nle, Sar, Tic |

Die Exendin Analoga, welche Gegenstand dieser Erfindung sind, besitzen vorteilhafte therapeutische Eigenschaften. So führen sie zu einer Stimulation der Insulinfreisetzung aus dem endokrinen Pankreas, zu einer Erhöhung der Insulinbiosynthese sowie zu einer vermehrten peripheren Glukose-Utilisation. Da diese Effekte nur bei gleichzeitig erhöhten Blutzuckerspiegeln zu beobachten sind, ist nach ihrer Verabreichung nicht mit dem Auftreten einer Hypoglykämie zu rechnen. Weiterhin hemmen die Exendin-Analoga die Glukaganfteisetzung aus dem endokrinen Pankreas und fuhren so zu einer Absenkung der Glukoneogenese. Die Exendin-Analoga führen beim nichtinsulinabhängigen Diabetes mellitus (NTDDM) zu einer deutlichen Verbesserung der Stoffwechselsituation. Insbesondere wird unabhängig von der Insulin-sekretorischen Wirkung die Glukoseaufnahme in Muskel- und Fettgewebe gesteigert. Aufgrund der inhibitorischen Wirkung auf die Glukagonfreisetzung ist auch die Verabreichung der Exendin-Analoga beim insulinabhängigen Diabetes mellitus sinnvoll. Gegenüber Glucagon-like peptide-1 (GLP-1) und den bekannten Exendin-3 und Exendin-4 Sequenzen besitzen die erfindungsgemäßen Exendin-Analoga eine überraschend höhere Wirksamkeit in den verschiedenen Testsystemen, so daß sie für eine therapeutische Anwendung besser geeignet sind als GLP-1, Exendin-3 oder Exendin-4. Die Vorteile der neuen Exendin-Analoga sind insbesondere die folgenden: höhere Stabilität gegenüber Abbau und Metabolisierung, längere Wirkdauer, Wirksamkeit bei niedrigeren Dosen. Besondere bevorzugt sind Analoga auf Basis von Exendin-3, die besonders lange Wirkdauern oder Wirksamkeit bei besonders niedriger Dosis zeigen.

Die Festphasen- und Flüssigphasensynthese ist ein übliches Verfahren zur Herstellung von Peptiden. Um das Verfahren für die Herstellung eines bestimmten Produktes im Hinblick auf die Reinheit des Rohproduktes und die Ausbeute zu optimieren, ist es erforderlich, die Prozeßparameter und die verwendeten Materialien, beispielsweise das Trägermaterial, die Reagenzien, welche Gruppen zur Reaktion bringen sollen, die Materialien für das Blockieren der Gruppen, welche nicht reagieren sollen, oder die Reagenzien, welche blockierende Materialien abspalten, an das herzustellende Produkt, an die herzustellenden Zwischenprodukte bzw. Ausgangsmaterialien anzupassen. Diese Anpassung ist angesichts der Interpendenz der vielen Verfahrensparameter nicht einfach.

Arzneimittel, welche die erfindungsgemäßen Peptide einzeln oder zusammen als aktiven Wirkstoff neben üblichen Hilfs-und Zusatzstoffen enthalten, werden vorzugsweise parenteral (subcutan, intramuskulär oder intravenös) verabreicht. In Frage kommen aber auch alle sonst üblichen Applikationsverfahren wie oral, rectal, buccal (einschließlich sublingual), pulmonal, transdermal, iontophoretisch, vaginal und intranasal. Das Arzneimittel hat eine insulinregulierende Wirkung und fördert dabei in vorteilhafter Weise den Ausgleich des Blutzuckerspiegels. Vorteilhaft fiir die Anwendung des Arzneimittels ist es, wenn Blutspiegel zwischen 20 und 50 pmol/l erreicht werden. Dazu sind Infusionsraten von 0,4 -1,2 pmol/kg/Min. erforderlich. Bei subcutaner bzw buccaler Applikation sind je nach galenischer Form und angestrebter Wirkdauer Substanzmengen von 5 - 500 nmol erforderlich.

Die erfindungsgemäßen Exendin-Analoga oder pharmakologisch verträglichen Salze hiervon werden vorzugsweise als sterile Lyophilisate gelagert und vor der Applikation mit einer geeigneten isotonischen Lösung vermischt. In dieser Form können die Analoga dann injiziert, infundiert oder gegebenenfalls auch durch die Schleimhäute absorbiert werden. Als Lösungsmittel können die üblichen, für die Injektion oder Infusion geeigneten isotonischen, wässrigen Systeme, die die bei Injektionslösungen üblichen Zusätze wie Stabilisierungsmittel und Lösungsvermittler enthalten, verwendet werden. Physiologische Kochsalzlösung oder gegebenenfalls durch Puffer isotonisch gestellte Lösungen werden in diesem Fall bevorzugt.

Zusätze sind z. B. Tartrat- oder Citratpuffer, Ethanol, Komplexbildner (wie Ethylendianmintetraessigsäure und deren nichttoxischen Salze), hochmolekulare Polymere (wie flüssiges Polyethylenoxid) zur Viskositätsregelung. Flüssige Trägerstoffe für Injektionslösungen müssen steril sein und werden vorzugsweise in Ampullen abgefüllt. Feste Trägerstoffe sind z. B. Stärke, Lactose, Mannit, Methylcellulose, Talkum, hochdisperse Kieselsäuren, höhermolekulare Fettsäuren (wie Stearinsäure), Gelantine, Agar-Agar, Calciumphosphat, Magnesiumstearat, tierische und pflanzliche Fette, feste hochmolekulare Polymere (wie Polyethylenglykol); für orale Applikation geeignete Zubereiteungen können falls gewünscht Geschmacks- und Süßstoffe enthalten. Für die nasale Applikation können Surfactants zur Verbesserung der Absorption durch die nasale Schleimhaut zugesetzt werden, z. B. Cholsäure, Taurocholsäure, Chenodeoxycholsäure, Glykolsäure, Dehydrocholsäure, Deoxycholsäure und Cyclodextrine.

Die zu verabreichende Tagesdosis liegt im Bereich von 150-500 nmol. Die Bestimmung der biologischen Aktivität basiert auf Messungen gegen internationale Referenzpräparationen für Glucagon-like peptide-1, Exendin-3 oder Exendin-4 in einem gebräuchlichen Testverfahren für Glucagon-like peptide-1.

Die erfindungsgemäßen Exendin-Analoga können nach den in der Peptidsynthese üblichen Verfahren hergestellt werden, wie sie z. B. in J. M. Steward und J. D. Young "Solid Phase Peptide Synthesis", 2nd ed., Pierce Chemical Co., Rockford, Illinois (1984) und J. Meienhofer "Hormonal Proteins and Peptides", Vol. 2, Academic Press, New York, (1973) für die Festphasensynthese und in E. Schroder und K. Lubke "The Peptides", Vol. 1, Academic Press, New York, (1965) für die Flüssigphasensynthese beschrieben worden sind.

## Allgemeine Verfahren zur Peptidsynthese

Im allgemeinen werden bei der Synthese von Peptiden geschützte Aminosäuren zu einer wachsenden Peptidkette addiert. Die erste Aminosäure ist entweder an der Aminogruppe oder der Carboxylgruppe sowie an jeglicher reaktiver Gruppe in der Seitenkette geschützt. Diese geschützte Aminosäure wird entweder an einen inerten Träger gekoppelt oder kann auch in Lösung eingesetzt werden. Die nächste Aminosäure in der Peptidsequenz wird passend geschützt unter Bedingungen, welche die Ausbildung einer Amidbindung begünstigen, zu der ersten gegeben. Nachdem alle gewünschten Aminosäuren in der richtigen sequentiellen Abfolge gekuppelt wurden, werden die Schutzgruppen und gegebenenfalls die Trägerphase abgespalten. Das erhaltene rohe Polypeptid wird umgefällt und vorzugsweise chromatographisch zum Endprodukt gereinigt.

Eine bevorzugte Methode zur Darstellung von Analoga physiologisch aktiver Polypeptide, mit weniger als etwa vierzig Aminosäuren, beinhaltet die Festphasenpeptidsynthese. Bei dieser Methode werden die $\alpha$-Aminofunktionen ($N^{\alpha}$) und jegliche reaktive Seitenketten mit säure- oder basenlabilen Gruppen geschützt. Die verwendeten Schutzgruppen sollten unter den Bedingungen der Knüpfung von Amidbindungen stabil sein, aber sich leicht abspalten lassen ohne die entstandene Polypeptidkette zu beeinträchtigen. Zu den geeigneten Schutzgruppen für die $\alpha$-Aminofunktion gehören die folgenden Gruppen, sind aber nicht auf diese limitiert: t-Butyloxycarbonyl (Boc), Benzyloxycarbonyl (Z), o-Chlorbenzyloxycarbonyl, Biphenylisopropyloxycarbonyl, *tert.*-Amyloxycarbonyl (Amoc), $\alpha,\alpha$-Dimetyl-3,5-dimethoxybenzyloxycarbonyl, o-Nitrosulfenyl, 2-Cyano-t-butoxycarbonyl, 9-Fluorenylmethoxycarbonyl (Fmoc), 1-(4,4-dimethyl-2,6-dioxocylohex-1-yliden)ethyl (Dde) und ähnliche. Vorzugsweise wird 9-Fluorenylmethoxycarbonyl (Fmoc) als $N^{\alpha}$-Schutzgruppen eingesetzt.

Zu den geeigneten Seitenkettenschutzgruppen gehören die folgenden, sind aber nicht auf diese limitiert: Acetyl, Allyl (All), Allyloxycarbonyl (Alloc), Benzyl (Bzl), Benzyloxycarbonyl (Z), t-Butyloxycarbonyl (Boc), Benzyloxymethyl (Bom), o-Brombenzyloxycarbonyl, t-Butyl (tBu), t-Butyldimethylsilyl, 2-Chlorbenzyl, 2-Chlorbenzyloxycarbonyl (2-ClZ), 2,6-Dichlorbenzyl, Cyclohexyl, Cyclopentyl, 1-(4,4-dimethyl-2,6-dioxocylohex-1-yliden)ethyl (Dde), Isopropyl, 4-Methoxy-2,3,6-trimethylbenzyl-sulfonyl (Mtr), 2,2,5,7,8 Pentamethylchroman-6-sulfonyl (Pmc), Pivalyl, Tetrahydropyran-2-yl, Tosyl (Tos), 2,4,6-Trimethoxybenzyl, Trimethylsilyl und Trityl (Trt).

Bei der Festphasensynthese wird die C-terminale Amionosäure als erste an ein geeignetes Trägermaterial gekuppelt. Geeignete Trägermaterialien sind solche, die inert gegen die Reagenzien und die Reaktionsbedingungen der schrittweisen Kondensations- und Abspaltungsraktionen sind, und welche sich nicht in den benützten Reaktionsmedien lösen. Beispiele für kommerziell erhältliche Trägermaterialien beinhalten Styrol/Divinylbenzol Copolymerisate, welche mit reaktiven Gruppen und/oder Polyethylenglykol modifiziert wurden, so auch chlormethyliertes Styrol/Divinylbenzol Copolymer, hydroxy- oder aminomethyliertes Styrol/Divinylbenzol Copolymer und ähnliche. Mit 4-Benzyloxybenzylalkohol (Wang-Anker (Wang, S. S. 1973)) oder 2-Chlortritylchlorid (Barlos, K. et. al. 1989) derivatisiertes Polystyrol(1%)divinylbenzol oder TentaGel® (Rapp Polymere, Tübingen) wird bevorzugt eingesetzt, falls die Peptidsäure dargestellt werden soll. Handelt es sich um das Peptidamid, so wird das mit 5-(4'-aminomethyl)-3',5'-dimethoxy-phenoxy)valeriansäure (PAL-Anker) (Albericio, F. et. al. 1987) oder der p-(2,4-Dimetho%Whenyl-aminomethyl)-phenon-Gruppe (Rink-Amid-Anker (Rink, H. 1987)) derivatisiertes Polystyrol(1%)divinylbenzol oder TentaGel® bevorzugt.

Die Anknüpfung an die polymeren Träger kann durch Reaktion der C-terminalen Fmocgeschützten Aminosäure, unter Zusatz eines Aktivierungsreagenzes, in Ethanol, Acetonitril, N,N-Dimethylformamid (DMF), Dichlormethan, Tetrahydrofuran, N-Methylpyrrolidon oder ähnlichen Solventien, vorzugsweise in DMF, mit dem Trägermaterial bei Raumtemperatur oder erhöhten Temperaturen, z.B. zwischen 40°C und 60°C, vorzugsweise bei Raumtemperatur, und Reaktionszeiten von 2 bis 72 Stunden, vorzugsweise etwa 2 x 2 Stunden, erreicht werden.

Die Kupplung der $N^\alpha$-geschützten Aminosäure, vorzugsweise der Fmoc-Aminosäure, an das PAL-, Wang- oder Rink-Anker kann beispielsweise mit Hilfe von Kupplungsreagenzien wie N,N'-Dicyclohexylcarbodiimid (DCC), N,N'-Diisopropylcarbodiimid (DIC) oder anderen Carbodiimiden, 2-(1H-Benzotriazol-1-yl)-1,1,3,3-tetramethyluronium tetrafluoroborat (TBTU) oder anderen Uronium-Salzen, O-Acyl-Harnstoffen, Benzotriazol-1-yl-oxy-tris-pyrrolidino-phosphonium hexafluorophosphat (PyBOP) oder anderen Phosphonium-Salzen, N-hydroxysuccinimiden, anderen N-Hydroxyimiden, oder Oximen, in Gegenwart oder auch in Abwesenheit von 1-Hydroxybenzotriazol oder 1-Hydroxy-7-azabenzotriazol, vorzugsweise mit Hilfe von TBTU unter Zusatz von HOBt, mit oder ohne Zusatz einer Base wie beispielsweise Diisopropylethylamin (DIEA), Triethylamin oder N-Methylmorpholin, vorzugsweise Diisopropylethylamin, bei Reaktionszeiten von 2 bis 72 Stunden, vorzugsweise 3 Stunden, in einem 1,5 bis 3 fachem Überschuß der Aminosäure und der Kupplungsreagenzien, vorzugsweise in einem 2fachen Überschuß und Temperaturen zwischen etwa 10°C und 50°C, vorzugsweise bei 25°C, in einem Lösungsmittel wie Dimethylformamid, N-Methylpyrrolidon oder Dichlormethan, vorzugsweise Dimethylformamid, durchgeführt werden. Anstelle der Kupplungseagenzien kann auch der Aktivester (z.B. Pentafluorphenyl, p-Nitrophenyl oder ähnliche), das symmetrische Anhydrid der $N^\alpha$-Fmoc-Aminosäure, deren Säurechlorid oder -fluorid unter den oben beschriebenen Bedingungen eingesetzt werden.

Die Kupplung der $N^\alpha$-geschützten Aminosäure, vorzugsweise der Fmoc-Aminosäure, an das 2-Chlortrityl-Harz wird bevorzugt in Dichlormethan unter Zusatz von DIEA, bei Reaktionszeiten von 10 bis 120 Minuten, vorzugsweise 20 Minuten, durchgeführt, ist aber nicht auf die Verwendung dieses Lösungsmittels und dieser Base beschränkt.

Die sukzessive Kupplung der geschützten Aminosäuren kann nach den in der Peptidsynthese üblichen Verfahren typischerweise in einem Peptidsyntheseautomaten durchgeführt werden. Nach Abspaltung der $N^\alpha$-Fmoc-Schutzgruppe der gekuppelten Aminosäure auf der Festphase durch Behandlung mit Piperidin (10% bis 50%) in Dimethylformamid für 5 bis 20 Minuten, vorzugsweise 2x2 Minuten mit 50% Piperidin in DMF und 1 x 15 Minuten mit 20% Piperidin in DMF, wird die nächste geschützte Aminosäure in einem 3 bis 10 fachem Überschuß, vorzugsweise in einem 10fachen Überschuß, in einem inerten, nichtwässrigen, polaren Lösungsmittel, wie Dichlormethan, DMF oder Mischungen aus beiden, vorzugsweise DMF, und Temperaturen zwischen etwa 10°C und 50°C, vorzugsweise bei 25°C, an die vorhergehende gekoppelt. Als Kupptungsreagenzien kommen die bei der Kupplung der ersten $N^\alpha$-Fmoc-Aminosäure an den PAL-, Wang- bzw. Rink-Anker bereits erwähnten Reagenzien in Frage. Wiederum können alternativ auch Aktivester der geschützten Aminosäure deren Chloride oder Fluoride oder deren symmetrische Anhydride verwendet werden.

Am Ende der Festphasensynthese wird das Peptid vom Trägermaterial abgespalten unter gleichzeitiger Abspaltung der Seitenkettenschutzgruppen: Die Abspaltung kann mit Trifluoressigsäure oder anderen stark sauren Medien unter Zusatz von 5% - 20% v/v Scavengern wie Dimethylsulfid, Ethylmethylsulfid, Thioanisol, Thiokresol, m-Kresol, Anisol Ethandithiol, Phenol oder Wasser, vorzugsweise 15% v/v Dimethylsulfid/ Ethandithiol/ m-Kresol 1.1:1, innerhalb von 0,5 bis 3 Stunden, vorzugsweise 2 Stunden, erfolgen. In den Seitenkettten vollgeschützte Peptide werden durch Spaltung des 2-Chlortritylankers mit Eis-essig/Trifluorethanol/Dichlormethan 2:2:6 erhalten. Das geschützte Peptid kann durch Chromatographie über Silicagel gereinigt werden. Ist das Peptid über den Wang-Anker mit der Festphase verbunden, und soll ein Peptid mit C-terminaler Alkylamidierung erhalten werden, so kann die Abspaltung über eine Aminolyse mit einem Alkylamin oder Fluoroalkylamin durchgeführt werden. Die Aminolyse wird bei Temperaturen zwischen etwa -10°C und 50°C, vorzugsweise etwa 25°C, und Reaktionszeiten zwischen etwa 12 und 24 Stunden, vorzugsweise etwa 18 Stunden, durchgeführt. Weiterhin kann das Peptid auch durch Umesterung , z. B. mit Methanol, vom Träger gespalten werden.

Die erhaltene saure Lösung wird mit der 3- 20 fachen Menge an kaltem Ether oder n-Hexan, vorzugsweise einem 10fachen Überschuß Diethylether versetzt, um das Peptid auszufällen und damit von den im Ether verbleibenden Scavengern und abgespaltenen Schutzgruppen abzutrennen. Eine weitere Reinigung kann durch mehrfaches Umfällen des Peptides aus Eis-essig erfolgen. Das erhaltene Precipitat wird in Wasser oder tert.Butanol oder Mischungen der beiden Lösungsmittel, vorzugsweise einer 1:1 Mischung von tert Butanol/Wasser, aufgenommen und gefriergetrocknet.

Das erhaltene Peptid kann durch einzelne oder alle der folgenden chromatographischen Methoden gereinigt werden: Ionenaustausch über ein schwach basisches Harz in der Acetat Form; hydrophobe Adsorptionschromatographie an nicht derivatisierten Polystyrol/Divinylbenzol-Copolymeren (z.B. Amberlite® XAD); Adsorptionschromatographie an Silicagel; Ionenaustauschchromatographie an Carboxymethylcellulose; Verteilungschromatographie, z.B. an Sephadex® G-25; Gegenstromverteilungschromatographie; oder Hochdruckflüssigkeitschromatographie (HPLC), insbesondere "reversed-phase" HPLC an Octyl- oder Octadecylsilylsilica (ODS) -Phasen.

Zusammenfassend beinhaltet ein Teil der vorliegenden Erfindung Verfahren zur Darstellung von Polypeptiden, und deren pharmazeutisch verwendbaren Salzen. Diese Verfahren, welche zu physiologisch aktiven verkürzten Homologen und Analogen von Exendin-3 oder Exendin-4; mit den oben erwähnten bevorzugten Kettenlängen und Modifikationen führen, setzen sich aus Verfahren zur sequentiellen Kondensation geschützter Aminosäuren auf einem geeigneten Trägermaterial, zur Abspaltung des Trägers und der Schutzgruppen, und zur Reinigung der erhaltenen Rohpeptide zusammen.

Die Aminosäurenanalyse wurde mit einem Anünosäurenanalysator 420A der Firma Applied Biosystems (Weiterstadt) durchgeführt. 50 bis 1000 pmol der zu analysierenden Probe wurden in 10 bis 40 µl Lösung auf den Probenträger aufgetragen und anschließend vollautomatisch in der Gasphase bei 160°C mit 6N Salzsäure 90 Minuten hydrolysiert, mit Phenylisothiocyanat derivatisiert und on-line über eine Microbore-HPLC analysiert. Massenspektroskopische Untersuchungen wurden wurden an einem API III Triple-Quadrupol-Massenspektrometer (SCIEX, Thomhill, Kanada) ausgerüstet mit Ionenspray Ionenquelle durchgeführt.

Die geschützten Aminosäurenderivate können z.B. von der Novabiochem GmbH (Bad Soden) bezogen werden.

Die folgenden Beispiele stellen nur eine illustrierende Auswahl des Erfindungsgedanken dar und keine Einschränkung des Erfindungsgegenstandes.

**Beispiel 1**

HGEGTFTSDLSKQ-Nle-EEEAVRLFIEWLKNGR-$NH_2$ (SEQ ID Nr. 3) [Nle$^{14}$, Arg$^{30}$]-Exendin-4-(1-30)-$NH_2$

Beispiel 1 wurde in einem 0,02 mmol Ansatz nach der Festphasenmethode auf 5-(4'-aminomethyl)-3',5'-dimethoxyphenoxy)valerianyl-alanyl-aminomethylpolystyrol(1%)divinylbenzol (Beladung: 0,5 mmol/g) auf einem Multiplen Peptidsyntheseautomaten SyRo II der Firma MultiSynTech (Bochum) synthetisiert. Die α-Aminofunktionen der Aminosäuren waren 9-Fluorenylmethoxycarbonyl (Fmoc) geschützt. Als Seitenkettenschutzgruppen wurden t-Butyl (tBu) für Asp, Glu, Ser und Thr, Trityl (Trt) für Asn, Gln und His, t-Butyloxycarbonyl (Boc) für Lys und Trp und 2,2,5,7,8-Pentamethylchroman-6-sulfonyl (Pmc) für Arg eingesetzt. Die sequentielle Kupplung der geschützten Aminosäuren erfolgte in 10fachem Überschuß mit Doppelkupplungen von 2 mal 40 Minuten Dauer und mit N,N-Diisopropylcarbodiimid/1-Hydroxybenzotriazol als Aktivierungsreagenzien. Die Abspaltung des Peptides vom polymeren Träger unter gleichzeitiger Abspaltung der Schutzgruppen erfolgte in Trifluoressigsäure (85%) in Gegenwart von 15% Ethandithiol/Dimethylsulfid/m-Kresol (1:1:1 v/v/v) für 120 Minuten bei Raumtemperatur. Anschließend wurde das Peptid mit wasserfreiem Diethylether gefällt und zur vollständigen Entfernung der Thiole noch mehrfach mit wasserfreiem Diethyl-ether gewaschen. Gefriertrocknung des Precipitats aus Wasser/tert.-Butanol (1:1) ergab 62 mg des Rohpeptides. Das Rohpeptid wurde über reversed-phase HPLC mit einem Gradienten von 37% auf 42% Acetonitril/0,9% TFA in 30 Minuten gereinigt. Das Eluat wurde eingeengt, lyophilisiert und ergab eine Ausbeute von 29 mg eines weißen Feststoffes mit einer Reinheit von ≥ 97%.

Aminosäurenanalyse: Ala 1,08 (1); Asx 1,91 (2); Glx 6,10 (6); Phe 1,78 (2); Gly 3,10 (3); His 1,00 (1); Ile 0,88 (1); Lys 2,02 (2); Leu 3,24 (3); Nle 1,10 (1); Arg 1,98 (2); Ser 2,04 (2); Thr 1,99 (2); Val 0,91 (1); Trp 0,87 (1).

ESI-MS: 3488,2

**Beispiel 2**

HGEGTFTSDLSKQ-Nle-EEEAVRLFIEWLKNGY-$NH_2$ (SEQ ID Nr. 4) [Nle$^{14}$, Tyr$^{30}$]-Exendin-4-(1-30)-$NH_2$

Beispiel 2 wurde in einem 0,0076 mmol Ansatz nach der Festphasenmethode auf TentaGel® (Rapp Polymere, Tübingen) synthetisiert, welches mit dem Rink-Amid-Anker (4-(2',4'-Dimethoxyphenyl-aminomethyl)-phenoxy-Gruppe) derivatisiert war (Beladung: 0, 18 mmol/g) auf einem Multiplen Peptidsyntheseautomaten SyRo II der Firma MultiSynTech (Bochum) synthetisiert. Die eingesetzten geschützten Aminosäuren waren analog zu Beispiel 1. Die sequentielle Kupplung der geschützten Aminosäuren erfolgte in 8fachem Überschuß mit Einfachkupplungen von 40 Minuten Dauer, bei 40°C und unter Rühren. Als Aktivierungsreagenzien wurden 2-(1H-Benzotriazol-1-yl)-1,1,3,3-tetramethyluronium tetrafluoroborat (TBTU)/1-Hydroxybenzotriazol unter Zusatz von Diisopropylethylamin verwendet. Die Abspaltung und Aufreinigung des Peptides erfolgte analog zu Beispiel 1. Es wurden 18,1 mg eines weißen Feststoffes mit einer Reinheit von ≥ 95% erhalten.

Aminosäurenanalyse: Ala 1,03 (1); Asx 1,90 (2); Glx 6,24 (6); Phe 1,94 (2); Gly 3,12 (3); His 1,02 (1); Ile 1,09 (1); Lys 2,01 (2); Leu 3,06 (3); Nie 1,08 (1); Arg 0,97 (1); Ser 1,98 (2); Thr 1,80 (2); Val 0,93 (1); Trp 1,01 (1); Tyr 0,90 (1).

ESI-MS: 3494,8

**Beispiel 3**

HSDGTFTSDLSKQ-Nle-EEEAVRLFIEWLKNGR-$NH_2$ (SEQ ID Nr. 5) [Nle$^{14}$, Arg$^{30}$]-Exendin-3-(1-30)-$NH_2$

Beispiel 3 wurde analog nach der für Beispiel 2 beschriebenen Methode synthetisiert. Es wurden 17,6 mg eines weißen Feststoffes mit einer Reinheit von ≥ 99% erhalten.

Aminosäurenanalyse: Ala 0,99 (1); Asx 2,98 (3); Glx 5, 16 (5); Phe 2,08 (2); Gly 2,16 (2); His 0,95 (1); Ile 1,03 (1); Lys 2,04 (2); Leu 2,91 (3); Nle 1,05 (1); Arg 1,04 (1); Ser 3,00 (3); Thr 2,05 (2); Val 1,01 (1); Trp 1,18 (1); Tyr 0,98 (1).

ESI-MS: 3504,4

**Beispiel 4**

HGEGTFTSDLSKQMEEEAVRLFIEWLKNGR-NH$_2$ (SEQ ID Nr: 6) [Arg$^{30}$]-Exendin-4-(1-30)-NH$_2$

Beispiel 4 wurde analog nach der für Beispiel 1 beschriebenen Methode synthetisiert. Es wurden 17,9 mg eines weißen Feststoffes mit einer Reinheit von ≥ 96% erhalten.
Aminosäurenanalyse: Ala 0,96 (1); Asx 2,01 (2); Glx 6,00 (6); Phe 1,80 (2); Gly 3,21 (3); His 0,96 (1); Ile 1,07 (1); Lys 1,92 (2); Leu 2,98 (3); Met 1,06 (1); Arg 1,90 (2); Ser 1,91 (2); Thr 2,09 (2); Val 0,97 (1); Trp 0,84 (1).
ESI-MS: 3508,4

**Beispiel 5**

GEGTFTSDLSKQ-Nle-EEEAVRLFIEWLKNGR-NH$_2$ (SEQ ID Nr. 7) [Nle$^{14}$, Arg$^{30}$]-Exendin-4-(2-30)-NH$_2$

Beispiel 5 wurde analog nach der für Beispiel 2 beschriebenen Methode synthetisiert. Es wurden 13,2 mg eines weißen Feststoffes mit einer Reinheit von ≥ 97% erhalten.
Aminosäurenanalyse: Ala 1,04 (1); Asx 1,98 (2); Glx 6,08 (6); Phe 1,86 (2); Gly 2,91 (3); Ile 0,96 (1); Lys 1,84 (2); Leu 2,98 (3); Nle 1,04 (1); Arg 1,90 (2); Ser 1,94 (2); Thr 1,92 (2); Val 0,96 (1); Trp 0,85 (1). ESI-MS: 3350,8

**Beispiel 6**

HGEGTFTSDLSKQMEEEAVRAFIEWLKNGR-NH$_2$ (SEQ ID Nr. 8) [Ala$^{21}$, Arg$^{30}$]-Exendin-4-(1-30)-NH$_2$

Beispiel 6 wurde analog nach der für Beispiel 1 beschriebenen Methode synthetisiert. Es wurden 11,1 mg eines weißen Feststoffes mit einer Reinheit von ≥ 95% erhalten.
Aminosäurenanalyse: Ala 2,08 (2); Asx 1,93 (2); Glx 6,07 (6); Phe 1,74 (2); Gly 2,97 (3); His 0,98 (1); Ile 0,87 (1); Lys 2,15 (2); Leu 2,02 (2); Met 0,96 (1); Arg 2,13 (2); Ser 1,87 (2); Thr 2,07 (2); Val 1,04 (1); Trp 0,87 (1).
ESI-MS: 3466,3

**Beispiel 7**

HGEGTFTSDLSKQMEEEA VRLFEWLKAGR-NH$_2$ (SEQ ID Nr. 9) [Ala$^{28}$, Arg$^{30}$]-Exendin-4-(1-30)-NH$_2$

Beispiel 7 wurde analog nach der für Beispiel 1 beschriebenen Methode synthetisiert. Es wurden 15,0 mg eines weißen Feststoffes mit einer Reinheit von ≥ 97% erhalten.
Aminosäurenanalyse: Ala 1,98 (2); Asx 0,98 (1); Glx 6,22 (6); Phe 1,92 (2); Gly 3,03 (3); His 0,99 (1); Ile 1,03 (1); Lys 2,05 (2); Leu 3,03 (3); Met 0,96 (1); Arg 1,84 (2); Ser 1,98 (2); Thr 2,09 (2); Val 1,01 (1); Trp 0,72 (1).
ESI-MS: 3465,4

**Beispiel 8**

HGEGTFTSDLSKQMEEEAVRAFIEWLKAGR-NH$_2$ (SEQ ID Nr. 10) [Ala$^{21.28}$, Arg$^{30}$]-Exendin-4-(1-30)-NH$_2$

Beispiel 8 wurde analog nach der für Beispiel 1 beschriebenen Methode synthetisiert. Es wurden 18,4 mg eines weißen Feststoffes mit einer Reinheit von ≥ 95% erhalten.
Aminosäurenanalyse: Ala 3,12 (3); Asx 0,99 (1); Glx 6,04 (6); Phe 1,80 (2); Gly 3,00 (3); His 0,96 (1); Ile 1,02 (1); Lys 1,84 (2); Leu 1,97 (2); Met 0,98 (1); Arg 2,03 (2); Ser 1,91 (2); Thr 1,88 (2); Val 0,99 (1); Trp 0,99 (1).
ESI-MS: 3423,3

**Beispiel 9**

In analoger Weise können die folgenden Exendinderivate in hoher Reinheit hergestellt werden. (Ex-4 = Exendin-4, Ex-3 = Exendin-3)

| Exendin-Derivat | Seq. | Sequenz |
|---|---|---|
| [A$^{14}$,R$^{30}$]-Ex-4-(1-30)-OH | 11 | HGEGTFTSDLSKQAEEEAVRLFIEWLK NGR-OH |
| Ac-[Ile$^{14}$,R$^{30}$]-Ex-4-(1-30)-NH$_2$ | 12 | Ac-HGEGTFTSDLSKQIleEEEAVRLFIEWLK NGR-NH$_2$ |
| [Nle$^{14}$]-Ex-4-(1-27)-NH$_2$ | 13 | HGEGTFTSDLSKQNleEEEAVRLFIEWL K-NH$_2$ |
| [A$^{14,29}$,R$^{30}$]-Ex-3-(1-30)-NH$_2$ | 14 | HSDGTFTSDLSKQAEEEAVRLFIEWLK NAR-NH$_2$ |
| [A$^{14,27}$,R$^{30}$]-Ex-3-(1-30)-NH$_2$ | 15 | HSDGTFTSDLSKQAEEEAVRLFIEWLA NGR-NH$_2$ |
| [A$^{14,26}$,R$^{30}$]-Ex-3-(1-30)-NH$_2$ | 16 | HSDGTFTSDLSKQAEEEAVRLFIEWAK NGR-NH$_2$ |
| [A$^2$, Nle$^{14}$,R$^{30}$]-Ex-4-(1-30)-NH$_2$ | 17 | HAEGTFTSDLSKQNleEEEAVRLFIEWL KNGR-NH$_2$ |
| [C$^2$, Nle$^{14}$,R$^{30}$]-Ex-4-(1-30)-NH$_2$ | 18 | HCEGTFTSDLSKQNleEEEAVRLFIEWL KNGR-NH$_2$ |
| [D$^2$, Nle$^{14}$,R$^{30}$]-Ex-4-(1-30)-NH$_2$ | 19 | HDEGTFTSDLSKQNleEEEAVRLFIEWL KNGR-NH$_2$ |
| [E$^2$, Nle$^{14}$,R$^{30}$]-Ex-4-(1-30)-NH$_2$ | 20 | HEEGTFTSDLSKQNleEEEAVRLFIEWL KNGR-NH$_2$ |
| [F$^2$, Nle$^{14}$,R$^{30}$]-Ex-4-(1-30)-NH$_2$ | 21 | HFEGTFTSDLSKQNleEEEAVRLFIEWLK NGR-NH$_2$ |
| [H$^2$ Nle$^{14}$,R$^{30}$]-Ex-4-(1-30)-NH$_2$ | 22 | HHEGTFTSDLSKQNleEEEAVRLFIEWL KNGR-NH$_2$ |

| | | |
|---|---|---|
| [I$^2$, Nle$^{14}$,R$^{30}$]-Ex-4-(1-30)-NH$_2$ | 23 | HIEGTFTSDLSKQNleEEEAVRLFIEWLK NGR-NH$_2$ |
| [K$^2$, Nle$^{14}$,R$^{30}$]-Ex-4-(1-30)-NH$_2$ | 24 | HKEGTFTSDLSKQNleEEEAVRLFIEWL KNGR-NH$_2$ |
| [L$^2$, Nle$^{14}$,R$^{30}$]-Ex-4-(1-30)-NH$_2$ | 25 | HLEGTFTSDLSKQNleEEEAVRLFIEWL KNGR-NH$_2$ |
| [M$^2$, Nle$^{14}$,R$^{30}$]-Ex-4-(1-30)-NH$_2$ | 26 | HMEGTFTSDLSKQNleEEEAVRLFIEWL KNGR-NH$_2$ |
| [N$^2$, Nle$^{14}$,R$^{30}$]-Ex-4-(1-30)-NH$_2$ | 27 | HNEGTFTSDLSKQNleEEEAVRLFIEWL KNGR-NH$_2$ |
| [P$^2$, Nle$^{14}$,R$^{30}$]-Ex-4-(1-30)-NH$_2$ | 28 | HPEGTFTSDLSKQNleEEEAVRLFIEWLK NGR-NH$_2$ |
| [Q$^2$, Nle$^{14}$,R$^{30}$]-Ex-4-(1-30)-NH$_2$ | 29 | HQEGTFTSDLSKQNleEEEAVRLFIEWL KNGR-NH$_2$ |
| [R$^2$, Nle$^{14}$,R$^{30}$]-Ex-4-(1-30)-NH$_2$ | 30 | HREGTFTSDLSKQNleEEEAVRLFIEWL KNGR-NH$_2$ |
| [S$^2$, Nle$^{14}$,R$^{30}$]-Ex-4-(1-30)-NH$_2$ | 31 | HSEGTFTSDLSKQNleEEEAVRLFIEWLK NGR-NH$_2$ |
| [T$^2$, Nle$^{14}$,R$^{30}$]-Ex-4-(1-30)-NH$_2$ | 32 | HTEGTFTSDLSKQNleEEEAVRLFIEWL KNGR-NH$_2$ |
| [V$^2$, Nle$^{14}$,R$^{30}$]-Ex-4-(1-30)-NH$_2$ | 33 | HVEGTFTSDLSKQNleEEEAVRLFIEWL KNGR-NH$_2$ |
| [W$^2$, Nle$^{14}$,R$^{30}$]-Ex-4-(1-30)-NH$_2$ | 34 | HWEGTFTSDLSKQNleEEEAVRLFIEWL KNGR-NH$_2$ |
| [Y$^2$, Nle$^{14}$,R$^{30}$]-Ex-4-(1-30)-NH$_2$ | 35 | HYEGTFTSDLSKQNleEEEAVRLFIEWL KNGR-NH$_2$ |
| [A$^{2,24}$,G$^{16}$,E$^{21}$,K$^{20,28}$,Q$^{17}$,R$^{30}$,S$^{12}$, V$^{27}$,Y$^{13}$]-Ex-3-(1-30)-NH$_2$ | 36 | HADGTFTSDLSSYMEGQAVKEFIAWL VKGR-NH$_2$ |
| [A$^{14,25}$,R$^{30}$]-Ex-3-(1-30)-NH$_2$ | 37 | HSDGTFTSDLSKQAEEEAVRLFIEALKN GR-NH$_2$ |

| | | |
|---|---|---|
| $[E^3, A^{14}, R^{30}]$-Ex-3-(1-30)-NH$_2$ | 38 | HSEGTFTSDLSKQAEEEAVRLFIEWLKN GR-NH$_2$ |
| $[A^1, V^{14}, R^{30}]$-Ex-3-(1-30)-NH$_2$ | 39 | ASDGTFTSDLSKQVEEEAVRLFIEWLK NGR-NH$_2$ |
| $[A^{3,14}, R^{30}]$-Ex-4-(1-30)-NH$_2$ | 40 | HGAGTFTSDLSKQAEEEAVRLFIEWLK NGR-NH$_2$ |
| $[A^{5,14}, R^{30}]$-Ex-4-(1-30)-NH$_2$ | 41 | HGEGAFTSDLSKQAEEEAVRLFIEWLK NGR-NH$_2$ |
| $[A^{14}, R^{30}, Y^5]$-Ex-4-(1-30)-NH$_2$ | 42 | HGEGYFTSDLSKQAEEEAVRLFIEWLK NGR-NH$_2$ |
| $[A^{14}, R^{30}, Y^6]$-Ex-4-(1-30)-NH$_2$ | 43 | HGEGTYTSDLSKQAEEEAVRLFIEWLK NGR-NH$_2$ |
| $[A^{14}, I^6, R^{30}]$-Ex-4-(1-30)-NH$_2$ | 44 | HGEGTITSDLSKQAEEEAVRLFIEWLKN GR-NH$_2$ |
| $[A^{14}, R^{30}, S^7]$-Ex-4-(1-30)-NH$_2$ | 45 | HGEGTFSSDLSKQAEEEAVRLFIEWLK NGR-NH$_2$ |
| $[A^{14}, R^{30}, Y^7]$-Ex-4-(1-30)-NH$_2$ | 46 | HGEGTFYSDLSKQAEEEAVRLFIEWLK NGR-NH$_2$ |
| $[A^{14}, R^{30}, T^8]$-Ex-4-(1-30)-NH$_2$ | 47 | HGEGTFTTDLSKQAEEEAVRLFIEWLK NGR-NH$_2$ |
| $[A^{14}, R^{30}, Y^8]$-Ex-4-(1-30)-NH$_2$ | 48 | HGEGTFTYDLSKQAEEEAVRLFIEWLK NGR-NH$_2$ |
| $[A^{14}, E^9, R^{30}]$-Ex-4-(1-30)-NH$_2$ | 49 | HGEGTFTSELSKQAEEEAVRLFIEWLK NGR-NH$_2$ |
| $[A^{10,14}, R^{30}]$-Ex-4-(1-30)-NH$_2$ | 50 | HGEGTFTSDASKQAEEEAVRLFIEWLK NGR-NH$_2$ |
| $[A^{11,14}, R^{30}]$-Ex-4-(1-30)-NH$_2$ | 51 | HGEGTFTSDLAKQAEEEAVRLFIEWLK NGR-NH$_2$ |
| $[A^{12,14}, R^{30}]$-Ex-4-(1-30)-NH$_2$ | 52 | HGEGTFTSDLSAQAEEEAVRLFIEWLK NGR-NH$_2$ |
| $[A^{13,14}, R^{30}]$-Ex-4-(1-30)-NH$_2$ | 53 | HGEGTFTSDLSKAAEEEAVRLFIEWLK |

NGR-NH$_2$

| | | |
|---|---|---|
| [A$^{14,15}$,R$^{30}$]-Ex-4-(1-30)-NH$_2$ | 54 | HGEGTFTSDLSKQAAEEAVRLFIEWLK NGR-NH$_2$ |
| [A$^{14,16}$,G$^1$,R$^{30}$,S$^5$]-Ex-3-(1-30)-NH$_2$ | 55 | GSDGSFTSDLSKQAEAEAVRLFIEWLK NGR-NH$_2$ |
| [A$^{14,17}$,K$^1$,R$^{30}$]-Ex-4-(1-30)-NH$_2$ | 56 | KGEGTFTSDLSKQAEEAAVRLFIEWLK NGR-NH$_2$ |
| [A$^{14}$,L$^{18}$,R$^{30}$]-Ex-4-(1-30)-NH$_2$ | 57 | HGEGTFTSDLSKQAEEELVRLFIEWLK NGR-NH$_2$ |
| [A$^{14}$,I$^{19}$,R$^{30}$]-Ex-4-(1-30)-NH$_2$ | 58 | HGEGTFTSDLSKQAEEEAIRLFIEWLKN GR-NH$_2$ |
| [A$^{14,20}$,R$^{30}$]-Ex-4-(1-30)-NH$_2$ | 59 | HGEGTFTSDLSKQAEEEAVALFIEWLK NGR-NH$_2$ |
| [A$^{14}$,R$^{30}$,Y$^{22}$]-Ex-3-(1-30)-NH$_2$ | 60 | HSDGTFTSDLSKQAEEEAVRLYIEWLK NGR-NH$_2$ |
| [A$^{14}$,R$^{30}$,V$^{23}$]-Ex-4-(1-30)-NH$_2$ | 61 | HGEGTFTSDLSKQAEEEAVRLFVEWLK NGR-NH$_2$ |
| [A$^{14}$,L$^{24}$,R$^{30}$]-Ex-4-(1-30)-NH$_2$ | 62 | HGEGTFTSDLSKQAEEEAVRLFILWLK NGR-NH$_2$ |
| [A$^{14,25}$,R$^{30}$]-Ex-4-(1-30)-NH$_2$ | 63 | HGEGTFTSDLSKQAEEEAVRLFIEALKN GR-NH$_2$ |
| [A$^{14,26}$,R$^{30}$]-Ex-4-(1-30)-NH$_2$ | 64 | HGEGTFTSDLSKQAEEEAVRLFIEWAK NGR-NH$_2$ |
| [A$^{14,27}$,R$^{30}$]-Ex-4-(1-30)-NH$_2$ | 65 | HGEGTFTSDLSKQAEEEAVRLFIEWLA NGR-NH$_2$ |
| [A$^{14,29}$,R$^{30}$]-Ex-4-(1-30)-NH$_2$ | 66 | HGEGTFTSDLSKQAEEEAVRLFIEWLK NAR-NH$_2$ |
| [A$^{14}$,R$^{30}$]-Ex-4-(1-30)-NH$_2$ | 67 | HGEGTFTSDLSKQAEEEAVRLFIEWLK NGR-NH$_2$ |
| [R$^{30}$]-Ex-3-(1-30)-NH$_2$ | 68 | HSDGTFTSDLSKQMEEEAVRLFIEWLK NGR-NH$_2$ |
| [Nle$^{14}$, Y$^{30}$]-Ex-3-(1-30)-NH$_2$ | 69 | HSDGTFTSDLSKQNleEEEAVRLFIEWL KNGY-NH$_2$ |

14

[Nle$^{14}$, R$^{30}$]-Ex-3-(1-30)-OH     70     HSDGTFTSDLSKQNleEEEAVRLFIEWL KNGR-OH

[A$^{14}$, R$^{30}$]-Ex-3-(1-30)-NH$_2$     71     HSDGTFTSDLSKQAEEEAVRLFIEWLK NGR-NH$_2$

[Nle$^{14}$, R$^{30}$]-Ex-3-(2-30)-NH$_2$     72     SDGTFTSDLSKQNleEEEAVRLFIEWLK NGR-NH$_2$

[Nle$^{14}$, R$^{30}$]-Ex-3-(3-30)-NH$_2$     73     DGTFTSDLSKQNleEEEAVRLFIEWLKN GR-NH$_2$

Ac-[Nle$^{14}$, R$^{30}$]-Ex-3-(2-30)-NH$_2$     74     Ac-SDGTFTSDLSKQNleEEEAVRLFIEWLK NGR-NH$_2$

Ac-[Nle$^{14}$, R$^{30}$]-Ex-3-(3-30)-NH$_2$     75     Ac-DGTFTSDLSKQNleEEEAVRLFIEWLKN GR-NH$_2$

[Nle$^{14}$]-Ex-3-(1-27)-NH$_2$     76     HSDGTFTSDLSKQNleEEEAVRLFIEWL K-NH$_2$

[K$^2$,P$^3$, A$^{14}$,R$^{30}$]-Ex-3-(1-30)-NH$_2$     77     HKPGTFTSDLSKQAEEEAVRLFIEWLK NGR-NH$_2$

[A$^2$, Nle$^{14}$,R$^{30}$]-Ex-3-(1-30)-NH$_2$     78     HADGTFTSDLSKQNleEEEAVRLFIEWL KNGR-NH$_2$

[C$^2$, Nle$^{14}$,R$^{30}$]-Ex-3-(1-30)-NH$_2$     79     HCDGTFTSDLSKQNleEEEAVRLFIEWL KNGR-NH$_2$

[D$^2$, Nle$^{14}$,R$^{30}$]-Ex-3-(1-30)-NH$_2$     80     HDDGTFTSDLSKQNleEEEAVRLFIEWL KNGR-NH$_2$

[E$^2$, Nle$^{14}$,R$^{30}$]-Ex-3-(1-30)-NH$_2$     81     HEDGTFTSDLSKQNleEEEAVRLFIEWL KNGR-NH$_2$

[F$^2$, Nle$^{14}$,R$^{30}$]-Ex-3-(1-30)-NH$_2$     82     HFDGTFTSDLSKQNleEEEAVRLFIEWL KNGR-NH$_2$

[G$^2$, Nle$^{14}$,R$^{30}$]-Ex-3-(1-30)-NH$_2$     83     HGDGTFTSDLSKQNleEEEAVRLFIEWL KNGR-NH$_2$

[H$^2$ Nle$^{14}$,R$^{30}$]-Ex-3-(1-30)-NH$_2$     84     HHDGTFTSDLSKQNleEEEAVRLFIEWL KNGR-NH$_2$

[I$^2$, Nle$^{14}$,R$^{30}$]-Ex-3-(1-30)-NH$_2$     85     HIDGTFTSDLSKQNleEEEAVRLFIEWLK

NGR-NH$_2$

| | | |
|---|---|---|
| [K$^2$, Nle14,R$^{30}$]-Ex-3-(1-30)-NH$_2$ | 86 | HKDGTFTSDLSKQNleEEEAVRLFIEWL KNGR-NH$_2$ |
| [L$^2$, Nle$^{14}$,R$^{30}$]-Ex-3-(1-30)-NH$_2$ | 87 | HLDGTFTSDLSKQNleEEEAVRLFIEWL KNGR-NH$_2$ |
| [M$^2$, Nle$^{14}$,R$^{30}$]-Ex-3-(1-30)-NH$_2$ | 88 | HMDGTFTSDLSKQNleEEEAVRLFIEWL KNGR-NH$_2$ |
| [N$^2$, Nle$^{14}$,R$^{30}$]-Ex-3-(1-30)-NH$_2$ | 89 | HNDGTFTSDLSKQNleEEEAVRLFIEWL KNGR-NH$_2$ |
| [P$^2$, Nle$^{14}$,R$^{30}$]-Ex-3-(1-30)-NH$_2$ | 90 | HPDGTFTSDLSKQNleEEEAVRLFIEWL KNGR-NH$_2$ |
| [Q$^2$, Nle$^{14}$,R$^{30}$]-Ex-3-(1-30)-NH$_2$ | 91 | HQDGTFTSDLSKQNleEEEAVRLFIEWL KNGR-NH$_2$ |
| [R$^2$, Nle$^{14}$,R$^{30}$]-Ex-3-(1-30)-NH$_2$ | 92 | HRDGTFTSDLSKQNleEEEAVRLFIEWL KNGR-NH$_2$ |
| [T$^2$, Nle$^{14}$,R$^{30}$]-Ex-3-(1-30)-NH$_2$ | 93 | HTDGTFTSDLSKQNleEEEAVRLFIEWL KNGR-NH$_2$ |
| [V$^2$, Nle$^{14}$,R$^{30}$]-Ex-3-(1-30)-NH$_2$ | 94 | HVDGTFTSDLSKQNleEEEAVRLFIEWL KNGR-NH$_2$ |
| [W$^2$, Nle$^{14}$,R$^{30}$]-Ex-3-(1-30)-NH$_2$ | 95 | HWDGTFTSDLSKQNleEEEAVRLFIEWL KNGR-NH$_2$ |
| [Y$^2$, Nle$^{14}$,R$^{30}$]-Ex-3-(1-30)-NH$_2$ | 96 | HYDGTFTSDLSKQNleEEEAVRLFIEWL KNGR-NH$_2$ |
| [A$^{3,14}$,R$^{30}$]-Ex-3-(1-30)-NH$_2$ | 97 | HSAGTFTSDLSKQAEEEAVRLFIEWLK NGR-NH$_2$ |
| [A$^{5,14}$,R$^{30}$]-Ex-3-(1-30)-NH$_2$ | 98 | HSDGAFTSDLSKQAEEEAVRLFIEWLK NGR-NH$_2$ |
| [A$^{14}$,R$^{30}$,Y$^5$]-Ex-3-(1-30)-NH$_2$ | 99 | HSDGYFTSDLSKQAEEEAVRLFIEWLK NGR-NH$_2$ |
| [A$^{14}$,R$^{30}$,Y$^6$]-Ex-3-(1-30)-NH$_2$ | 100 | HSDGTYTSDLSKQAEEEAVRLFIEWLK NGR-NH$_2$ |
| [A$^{14}$,I$^6$,R$^{30}$]-Ex-3-(1-30)-NH$_2$ | 101 | HSDGTITSDLSKQAEEEAVRLFIEWLKN GR-NH$_2$ |

[A$^{14}$,R$^{30}$,S$^7$]-Ex-3-(1-30)-NH$_2$    102    HSDGTFSSDLSKQAEEEAVRLFIEWLKN GR-NH$_2$

[A$^{14}$,R$^{30}$,Y$^7$]-Ex-3-(1-30)-NH$_2$    103    HSDGTFYSDLSKQAEEEAVRLFIEWLK NGR-NH$_2$

[A$^{14}$,R$^{30}$,T$^8$]-Ex-3-(1-30)-NH$_2$    104    HSDGTFTTDLSKQAEEEAVRLFIEWLK NGR-NH$_2$

[A$^{14}$,R$^{30}$,Y$^8$]-Ex-3-(1-30)-NH$_2$    105    HSDGTFTYDLSKQAEEEAVRLFIEWLK NGR-NH$_2$

[A$^{14}$,E$^9$,R$^{30}$]-Ex-3-(1-30)-NH$_2$    106    HSDGTFTSELSKQAEEEAVRLFIEWLKN GR-NH$_2$

[A$^{10,14}$,R$^{30}$]-Ex-3-(1-30)-NH$_2$    107    HSDGTFTSDASKQAEEEAVRLFIEWLK NGR-NH$_2$

[A$^{11,14}$,R$^{30}$]-Ex-3(1-30)-NH$_2$    108    HSDGTFTSDLAKQAEEEAVRLFIEWLK NGR-NH$_2$

[A$^{12,14}$,R$^{30}$]-Ex-3-(1-30)-NH$_2$    109    HGEGTFTSDLSAQAEEEAVRLFIEWLK NGR-NH$_2$

[A$^{13,14}$,R$^{30}$]-Ex-3-(1-30)-NH$_2$    110    HSDGTFTSDLSKAAEEEAVRLFIEWLK NGR-NH$_2$

[A$^{14,15}$,R$^{30}$]-Ex-3-(1-30)-NH$_2$    111    HSDGTFTSDLSKQAAEEAVRLFIEWLK NGR-NH$_2$

[A$^{14,17}$,K$^1$,R$^{30}$]-Ex-3-(1-30)-NH$_2$    112    KSDGTFTSDLSKQAEEAAVRLFIEWLK NGR-NH$_2$

[A$^{14}$,L$^{18}$,R$^{30}$]-Ex-3-(1-30)-NH$_2$    113    HSDGTFTSDLSKQAEEELVRLFIEWLK NGR-NH$_2$

[A$^{14}$,I$^{19}$,R$^{30}$]-Ex-3-(1-30)-NH$_2$    114    HSDGTFTSDLSKQAEEEAIRLFIEWLKN GR-NH$_2$

[A$^{14,20}$,R$^{30}$]-Ex-3-(1-30)-NH$_2$    115    HSDGTFTSDLSKQAEEEAVALFIEWLK NGR-NH$_2$

[A$^{14}$,R$^{30}$,Y$^{22}$]-Ex-3-(1-30)-NH$_2$    116    HSDGTFTSDLSKQAEEEAVRLYIEWLK NGR-NH$_2$

[A$^{14}$,R$^{30}$,V$^{23}$]-Ex-3-(1-30)-NH$_2$    117    HSDGTFTSDLSKQAEEEAVRLFVEWLK NGR-NH$_2$

[A$^{14}$,L$^{24}$,R$^{30}$]-Ex-3-(1-30)-NH$_2$     118     HSDGTFTSDLSKQAEEEAVRLFILWLK NGR-NH$_2$

Suc-[Nle$^{14}$, R$^{30}$]-Ex-3-(3-30)-NH$_2$     119     Suc-DGTFTSDLSKQNleEEEAVRLFIEWLKN GR-NH$_2$

[Nle$^{14}$, R$^{30}$]-Ex-3-(1-30)-NH$_2$     120     HSDGTFTSDLSKQNleEEEAVRLFIEWL KNGR-NH$_2$

Die folgenden Beispiele wurde in einem 0,02 mmol Ansatz nach der Festphasenmethode auf RAM-Harz® (Rapp Polymere, Tübingen) synthetisiert, bei dem Aminomethylpolystyrol(1%)divinylbenzol mit dem Rink-Amid-Anker (4-(2',4'-Dimethoxyphenylaminomethyl)-phenoxy-Gruppe) derivatisiert ist (Beladung: 0,5 mmol/g). Die Synthesen wurden auf einem Multiplen Peptidsyntheseautomaten SyRo II der Firma MultiSynTech (Bochum) durchgeführt. Die eingesetzten geschützten Aminosäuren waren analog zu Beispiel 1. Die sequentielle Kupplung der geschützten Aminosäuren erfolgte in 5fachem Überschuß mit Einfachkupplungen von 40 Minuten Dauer, bei 40°C und unter Rühren. Als Aktivierungsreagenzien wurde 2-(1H-Benzotriazol-1-yl)-1,1,3,3-tetramethyluronium tetrafluoroborat (TBTU) unter Zusatz von Diisopropylethylamin verwendet. Die Abspaltung und Aufreinigung des Peptides erfolgte analog zu Beispiel 1. In den folgenden beiden Tabellen sind die Ausbeuten, Reinheiten und Analysendaten der wie oben beschrieben synthetisierten Peptide aufgeführt.

**Tabelle 1**

| Seq. ID | Ausbeute [mg] | Reinheit [%] | ESI-MS |
| --- | --- | --- | --- |
| 36 | 6,0 | 99 | 3343,3 |
| 38 | 12,8 | 99 | 3477,4 |
| 68 | 14,2 | 99 | 3523,4 |
| 69 | 14,1 | > 99 | 3512,1 |
| 70 | 18,0 | 95 | 3506,0 |
| 71 | 22,4 | 95 | 3463,6 |
| 72 | 6,6 | > 99 | 3368,0 |
| 73 | 17,8 | > 99 | 3281,1 |
| 75 | 12,0 | 99 | 3323,1 |
| 76 | 14,0 | 99 | 3178,1 |
| 77 | 7,2 | 99 | 3486,6 |
| 78 | 23,8 | 95 | 3488,1 |
| 79 | 19,0 | 95 | 3520,1 |
| 80 | 15,2 | 95 | 3531,9 |
| 81 | 8,6 | > 99 | 3545,9 |
| 82 | 23,6 | 98 | 3563,9 |
| 83 | 25,4 | 95 | 3475,6 |
| 84 | 10,4 | > 99 | 3554,1 |
| 85 | 8,2 | > 99 | 3530,1 |
| 86 | 10,4 | 95 | 3545,3 |
| 87 | 9,2 | > 99 | 3530,1 |
| 88 | 13,6 | > 99 | 3548,1 |
| 89 | 10,6 | > 99 | 3531,0 |
| 90 | 9,4 | 96 | 3514,9 |
| 91 | 6,0 | > 99 | 3545,4 |
| 92 | 15,4 | > 99 | 3574,9 |
| 93 | 10,1 | > 99 | 3519,7 |
| 94 | 9,4 | > 99 | 3517,7 |

Tabelle fortgesetzt

| Seq. ID | Ausbeute [mg] | Reinheit [%] | ESI-MS |
|---------|---------------|--------------|--------|
| 95 | 12,0 | > 99 | 3604,7 |
| 96 | 9,8 | 95 | 3581,8 |
| 120 | 8,5 | 95 | 3505,6 |

Tabelle fortgesetzt

| Seq. ID | Ausbeute [mg] | Reinheit [%] | ESI-MS |
|---------|---------------|--------------|--------|

Tabelle 2

Aminosäurenanalysen

| Seq. ID | Ala | Arg | Asx | Glx | Gly | His | Ile | Leu | Lys | Nle | Phe | Ser | Thr | Trp | Val |
|---|---|---|---|---|---|---|---|---|---|---|---|---|---|---|---|
| 36[a] | 3,18 (3) | 0,99 (1) | 1,95 (2) | 3,04 (3) | 2,94 (3) | 0,89 (1) | 1,03 (1) | 2,14 (2) | 2,09 (2) | | 1,95 (2) | 3,06 (3) | 1,88 (2) | 0,99 (1) | 1,94 (2) |
| 38 | 2,08 (2) | 2,10 (2) | 2,00 (2) | 6,06 (6) | 2,04 (2) | 0,81 (1) | 1,01 (1) | 3,25 (3) | 2,13 (2) | | 1,96 (2) | 3,08 (3) | 1,82 (2) | 0,94 (1) | 1,02 (1) |
| 68[b] | 1,02 (1) | 2,14 (2) | 3,16 (3) | 5,06 (5) | 1,71 (2) | 0,93 (1) | 1,02 (1) | 3,21 (3) | 2,08 (2) | | 1,93 (2) | 3,00 (3) | 1,88 (2) | 0,98 (1) | 1,02 (1) |
| 69[c] | 1,02 (1) | 1,12 (1) | 2,99 (3) | 4,94 (5) | 1,81 (2) | 0,77 (1) | 1,12 (1) | 3,26 (3) | 2,12 (2) | 1,04 (1) | 1,99 (2) | 2,91 (3) | 1,81 (2) | 1,06 (1) | 1,03 (1) |
| 70 | 1,08 (1) | 2,15 (2) | 2,96 (3) | 5,07 (5) | 1,84 (2) | 0,85 (1) | 1,06 (1) | 3,23 (3) | 2,06 (2) | 0,92 (1) | 1,99 (2) | 2,01 (3) | 1,84 (2) | 1,05 (1) | 1,04 (1) |
| 71 | 1,98 (2) | 2,06 (2) | 3,14 (3) | 4,98 (5) | 1,95 (2) | 0,85 (1) | 1,01 (1) | 3,20 (3) | 2,08 (2) | | 1,94 (2) | 3,09 (3) | 1,90 (2) | 1,03 (1) | 0,96 (1) |
| 72 | 1,02 (1) | 2,12 (2) | 3,06 (3) | 5,02 (5) | 1,94 (2) | | 1,02 (1) | 3,25 (3) | 2,11 (2) | 0,95 (1) | 1,98 (2) | 2,92 (3) | 1,88 (2) | 1,02 (1) | 1,03 (1) |

EP 0 915 910 B1

EP 0 915 910 B1

| | | | | | | | | | | | | | | | |
|---|---|---|---|---|---|---|---|---|---|---|---|---|---|---|---|
| 73 | 1,01 (1) | 2,04 (2) | 3,02 (3) | 5,03 (5) | 1,83 (2) | | 0,99 (1) | 3,19 (3) | 2,07 (2) | 1,06 (1) | 1,98 (2) | 2,00 (2) | 1,87 (2) | 0,97 (1) | 1,03 (1) |
| 75 | 1,09 (1) | 2,21 (2) | 3,10 (3) | 5,18 (5) | 1,75 (2) | | 1,16 (1) | 3,26 (3) | 2,10 (2) | 1,08 (1) | 1,95 (2) | 2,10 (2) | 2,04 (2) | 1,28 (1) | 0,88 (1) |
| 76 | 1,03 (1) | 1,06 (1) | 2,05 (2) | 4,90 (5) | 0,94 (1) | 0,88 (1) | 1,03 (1) | 3,18 (3) | 2,01 (2) | 1,03 (1) | 1,95 (2) | 3,02 (3) | 1,86 (2) | 0,98 (1) | 1,03 (1) |
| 77[d] | 2,07 (2) | 2,00 (2) | 2,10 (2) | 5,07 (5) | 1,78 (2) | 0,93 (1) | 1,01 (1) | 3,14 (3) | 3,12 (3) | | 1,93 (2) | 2,05 (2) | 1,93 (2) | 1,01 (1) | 1,01 (1) |
| 78 | 2,11 (2) | 2,17 (2) | 3,14 (3) | 5,01 (5) | 1,92 (2) | 0,90 (1) | 1,06 (1) | 3,25 (3) | 2,06 (2) | 1,05 (1) | 1,99 (2) | 2,06 (2) | 1,90 (2) | 1,00 (1) | 1,05 (1) |
| 79[e] | 1,16 (1) | 2,10 (2) | 3,07 (3) | 5,07 (5) | 1,78 (2) | 0,66 (1) | 1,08 (1) | 3,30 (3) | 2,06 (2) | 0,96 (1) | 1,93 (2) | 2,02 (2) | 1,87 (2) | 1,06 (1) | 1,05 (1) |
| 80 | 1,07 (1) | 2,13 (2) | 3,78 (4) | 5,03 (5) | 1,82 (2) | 0,84 (1) | 1,05 (1) | 3,20 (3) | 2,04 (2) | 0,92 (1) | 1,97 (2) | 1,99 (2) | 1,82 (2) | 1,05 (1) | 1,03 (1) |
| 81 | 1,04 (1) | 2,06 (2) | 3,08 (3) | 5,96 (6) | 2,01 (2) | 0,82 (1) | 1,02 (1) | 3,17 (3) | 2,08 (2) | 0,95 (1) | 2,04 (2) | 2,05 (2) | 1,89 (2) | 1,01 (1) | 0,96 (1) |
| 82 | 1,09 (1) | 1,96 (2) | 3,01 (3) | 4,99 (5) | 1,89 (2) | 1,02 (1) | 1,02 (1) | 3,14 (3) | 2,04 (2) | 0,91 (1) | 2,74 (3) | 1,98 (2) | 1,85 (2) | 0,98 (1) | 0,99 (1) |
| 83 | 1,08 (1) | 2,22 (2) | 3,09 (3) | 5,01 (5) | 2,85 (3) | 0,83 (1) | 1,11 (1) | 3,23 (3) | 2,13 (2) | 1,12 (1) | 1,99 (2) | 2,09 (2) | 1,86 (2) | 0,99 (1) | 1,03 (1) |
| 84 | 1,01 (1) | 1,98 (2) | 3,09 (3) | 5,01 (5) | 1,71 (2) | 1,69 (2) | 1,07 (1) | 3,18 (3) | 2,07 (2) | 0,94 (1) | 1,99 (2) | 1,90 (2) | 1,84 (2) | 0,98 (1) | 1,01 (1) |
| 85 | 1,07 (1) | 2,12 (2) | 3,11 (3) | 5,03 (5) | 1,88 (2) | 0,93 (1) | 1,83 (2) | 3,23 (3) | 2,11 (2) | 1,11 (1) | 1,97 (2) | 2,03 (2) | 1,94 (2) | 0,97 (1) | 1,02 (1) |

| 86 | 87 | 88[f] | 89 | 90[g] | 91 | 92 | 93 | 94 | 95 | 96[h] | 120 |
|---|---|---|---|---|---|---|---|---|---|---|---|
| 0,99 (1) | 1,00 (1) | 1,02 (1) | 0,97 (1) | 0,97 (1) | 1,06 (1) | 1,03 (1) | 1,03 (1) | 1,72 (2) | 1,14 (1) | 1,05 (1) | 1,00 |
| 1,01 (1) | 0,95 (1) | 0,99 (1) | 0,99 (1) | 1,04 (1) | 1,11 (1) | 0,97 (1) | 0,95 (1) | 1,26 (1) | 1,76 (2) | 1,07 (1) | 0,99 |
| 1,88 (2) | 1,92 (2) | 1,88 (2) | 1,87 (2) | 1,92 (2) | 1,79 (2) | 1,87 (2) | 2,75 (3) | 2,00 (2) | 1,88 (2) | 1,84 (2) | 1,88 |
| 2,05 (2) | 2,07 (2) | 2,01 (2) | 2,03 (2) | 2,09 (2) | 1,95 (2) | 2,00 (2) | 2,06 (2) | 2,06 (2) | 2,02 (2) | 1,96 (2) | 2,94 |
| 1,97 (2) | 1,97 (2) | 1,93 (2) | 1,94 (2) | 1,96 (2) | 1,93 (2) | 1,97 (2) | 1,96 (2) | 1,92 (2) | 1,96 (2) | 2,02 (2) | 1,95 |
| 0,98 (1) | 0,93 (1) | 0,94 (1) | 0,92 (1) | 1,01 (1) | 1,05 (1) | 1,06 (1) | 1,00 (1) | 1,06 (1) | 0,95 (1) | 1,05 (1) | 0,93 |
| 3,00 (3) | 2,23 (2) | 2,09 (2) | 2,08 (2) | 2,10 (2) | 2,10 (2) | 2,07 (2) | 2,14 (2) | 2,06 (2) | 2,14 (2) | 2,15 (2) | 2,07 |
| 3,21 (3) | 4,05 (4) | 3,21 (3) | 3,19 (3) | 3,23 (3) | 3,21 (3) | 3,19 (3) | 3,28 (3) | 3,20 (3) | 3,31 (3) | 3,30 (3) | 3,18 |
| 1,11 (1) | 1,09 (1) | 1,02 (1) | 1,03 (1) | 1,02 (1) | 1,05 (1) | 0,99 (1) | 1,02 (1) | 1,14 (1) | 1,04 (1) | 1,14 (1) | 1,03 |
| 0,88 (1) | 0,89 (1) | 0,93 (1) | 0,88 (1) | 0,86 (1) | 0,80 (1) | 0,82 (1) | 0,82 (1) | 0,82 (1) | 0,80 (1) | 0,78 (1) | 1,03 |
| 2,10 (2) | 1,94 (2) | 1,71 (2) | 1,95 (2) | 1,97 (2) | 1,87 (2) | 1,83 (2) | 1,98 (2) | 1,72 (2) | 1,84 (2) | 1,84 (2) | 1,91 |
| 5,03 (5) | 5,03 (5) | 5,07 (5) | 5,06 (5) | 5,04 (5) | 5,73 (6) | 5,03 (5) | 5,04 (5) | 5,08 (5) | 5,04 (5) | 5,00 (5) | 5,06 |
| 3,07 (3) | 3,13 (3) | 3,16 (3) | 3,89 (4) | 3,18 (3) | 3,07 (3) | 3,02 (3) | 3,02 (3) | 3,04 (3) | 3,04 (3) | 3,03 (3) | 3,05 |
| 2,11 (2) | 2,16 (2) | 2,14 (2) | 1,97(2) | 2,09 (2) | 2,08 (2) | 3,11 (3) | 2,12 (2) | 2,17 (2) | 2,18 (2) | 2,25 (2) | 1,98 |
| 1,03 (1) | 1,11 (1) | 1,03 (1) | 1,08 (1) | 1,00 (1) | 1,04 (1) | 1,01 (1) | 1,05 (1) | 1,07 (1) | 1,05 (1) | 1,03 (1) | 1,11 |

22

EP 0 915 910 B1

(1)   (2)   (3)   (5)   (2)   (1)   (1)   (3)   (2)   (1)   (2)   (3)   (2)   (1)   (1)

[a]   Methionin 0,96 (1); Tyrosin 0,86 (1)

[b]   Methionin 0,94 (1)

[c]   Tyrosin 0,82 (1)

[d]   Prolin 1,02 (1)

[e]   Cystein konnte bei den gegebenen Hydrolysebedingungen nicht nachgewiesen werden.

[f]   Methinon 0,93 (1)

[g]   Prolin 0,86 (1)

[h]   Tyrosin 0,87 (1)

**Beispiel 10: Pharmakologische Daten**

**Peptidmetabolismus in Ektopeptidase-Preparationen oder an Nieren-Microvilli Membranpräparationen**

**Hintergrund**

Eine Gruppe von Ektopeptidasen ist verantwortlich für den post-sekretorischen Metabolismus von Peptidhormonen. Diese Enzyme sind an die Plasma-Membranen von verschiedenen Zelltypen gebunden. Ihre "active site" ist in Richtung des extrazellulären Raumes orientiert. Außerdem sind diese Enzyme in hohen Konzentrationen in den Bürstensaum Membranen der nierennahen Tubuli vorhanden. Nieren Bürstensaum Microvillimembranen (BBM) sind also eine gute Quelle für die relevanten Ektopeptidasen und können als in vitro Test für die metabolische Stabilität von synthetischen Peptiden eingesetzt werden. Alternativ können Ektopeptidase-Preparationen verwendet werden. Beispielhaft wurden die humane Neutrale Endopeptidase 24.11 sowie die Dipeptidyl Peptidase IV eingesetzt, da GLP-1 ein Substrat dieser beiden Ektopeptidasen ist.

**Präparation von Bürstensaum Microvillimembranen**

Mittels subzellulärer Fraktionierung unter Verwendung der Differentialzentrifugationsmethode (Booth and Kenny (1975)) werden Microvillimembranen des Ratten- und Schweinenierencortex isoliert. Zur Beurteilung des Reinheitsgrades und der Ausbeute der Membranen werden 4 Bürstensaum-Ektopeptidasen fluorimetrisch und andere Markerenzyme kolorimetrisch gemessen.

**Ektopeptidase Präparationen**

Gereinigte humane Neutrale Endopeptidase 24.11 wurde in der rekombinanten Form von Genentech (San Francisco, USA), Dipeptidyl Peptidase IV wurde als Isolat aus humaner Placenta von Calbiochem (Bad Soden) bezogen.

**Inkubations-Protokoll**

Microvilli Membranen (0,5 - 1 $\mu$g Protein) oder die jeweilige Ectopeptidase Präparation (60-300 ng) wurden mit 10 $\mu$g Peptid (etwa 3 nmol) in 100 $\mu$l HEPES Puffer (50 mM, pH 7,4), welcher 50 mM NaCl enthielt, inkubiert. An vorher bestimmten Zeitpunkten (Dauer bis zu 1 Stunde) wurden die Reaktionen durch Kochen abgebrochen. Anschließend wurden die Proben zentrifugiert (10.000 x g), mit 150 $\mu$l 0,1% TFA verdünnt und mittels "reversed phase" (RP) HPLC analysiert. Jede Probe wurde doppelt bestimmt.

**HPLC Analyse**

Für die HPLC Analyse wurde ein System mit den folgenden Komponenten verwendet: Eine "2248" Niederdruckpumpe (Pharmacia-LKB, Freiburg), ein WISP 10B Autoinjector (Millipore-Waters, Eschborn), ein UV-Detektor SP-4 (Gynkotec, Berlin), ein Niederdruck-Mischsystem (Pharmacia-LKB, Freiburg) und einer "Program Manager" Software-Steuerung (Pharmacia-LKB, Freiburg). Die Trennungen erfolgten über Lichrospher C-8, 5$\mu$, 4 $\times$ 124 mm (Merck, Darmstadt) mit einem binären Gradienten mit den Laufmitteln A: 0,1% Trifluoressigsäure (TFA) und B: Acetonitril:Wasser:TFA (70:29,9:0,1). Nach der Injektion von 244 $\mu$l der Probenlösung auf die mit Laufmittel A equilibrierte Säule, wurden die Inkubationsprodukte mit einem linearen Gradienten von 0% auf 80% B in 80 min eluiert und bei 215 nm UV-Absorption detektiert.

**Berechnung der Proteolyse-Raten**

Für jede Inkubationszeit eines jeden Peptides wurden zwei Messungen durchgeführt und die mittlere Peak-Höhe des Substrat-Peaks gegen die Zeit aufgetragen. Am Beispiel von GLP-1 konnte gezeigt werden, daß die Peakhöhe linear proportional zur Quantität des Peptides in der Probenlösung ist. Innerhalb der ersten Stunde der Inkubation mit den Microvilli-Membranen oder den Peptidasen konnte außerdem eine lineare Abnahme der Peakhöhe mit der Zeit beobachtet werden. Die Proteolyse-Rate wird also durch die Abnahme der Höhe des Substratpeaks bestimmt und in [$\mu$mol Substrat/mg Protein/Minute] angegeben.

**Abbaustabilität von Exendin-Analoga**

**Inkubation mit humaner Neutraler Endopeptidase 24.11b**

[Mle$^{14}$,Arg$^{30}$]-Exendin-4-(1-30)-NH$_2$ (Seq.ID Nr. 3) wurde mit der Neutralen Endopeptidase 24.11 wie oben beschrieben inkubiert und die Abbaurate wurde bestimmt. Als Kontrolle diente GLP1-(7-36)-NH$_2$. Die Ergebnisse sind in Tabelle 3 aufgeführt.

**Tabelle 3**

| Abbaurate [mM/100ng/ml NEP24.11/min] | |
|---|---|
| GLP1-(7-36)-NH$_2$ | 0,0586 |
| [Nle$^{14}$,Arg$^{30}$]-Ex-4-(1-30)-NH$_2$ $_{Bsp. 1}$ | 0,0083 |

**Inkubationen mit Dipeptidyl Peptidase IV**

Die in Tabelle 4 aufgeführten Peptide wurden mit Dipeptidyl Peptidase IV (DDP-IV) wie oben beschrieben inkubiert. Die Inkubation wurde jeweils zu dem Zeitpunkt abgebrochen, bei dem GLPI-(7-36)-NH$_2$ 50% Hydrolyse zeigte. Der Substratpeak jedes Peptides wurde aus dem rpHPLC-Lauf gesammelt und massenspektroskopisch untersucht, um trunkierte Produkte auszuschließen.

**Tabelle 4**

| Analogon | Seq.ID | Substrat für DDP-IV |
|---|---|---|
| [Al$^2$,Nle$^{14}$,Arg$^{30}$-Ex-3-(1-30)-NH$_2$ | 78 | keine Proteolyse |
| GLP1-(7-36)NH$_2$ | | 50% Proteolyse |

**Inkubationen mit Bürstensaum Microvillimembranen**

In Tabelle 5 sind die Proteolyseraten aufgeführt, welche nach Inkubation mit Bürstensaum Microvillimembranen (BBM) nach dem oben beschriebenen Protokoll berechnet wurden. Als Kontrolle dienten GLPI-(7-36)NH$_2$.

**Tabelle 5**

| Analogon | Seq.ID | Abbaurate [ng Peptid/min/mg BBM] |
|---|---|---|
| GLP1-(7-36)-NH$_2$ | | 880,00 |
| [Lys$^2$,Nle$^{14}$,Arg$^{30}$]-Ex-3-(1-30)-NH$_2$ | 86 | 2,05 |

**Insulinsekretion an isolierten Inselzellen**

**Organentnahme**

Den narkotisierten (0,3 - 0,5 ml Nembutal/isoton. Kochsalzlsg 1:4, i.p.) Mäusen wird durch einen Medianschnitt und zwei Flankenschnitte das Abdomen geöffnet, das Bauchfell fixiert und am Rippenbogen entlang das Zwerchfell aufgeschnitten. Durch Injektion einer Neutralrotlösung in die linke Herzkammer werden sämtliche Organe aufgebläht und rot angefärbt. Das Pankreas wird am Magen und am Duodenum entlang bis zu den Mesenterien vorsichtig abpräpariert. Bis zur Verdauung wird der Pankreas in einer eisgekühlten Petrischale in Hank's balanced salt solution (HBBS) und einigen Tropfen Neutralrot abgelegt.

**Inselpräparation**

Jeweils zwei Pankreas werden mit Zellstoff abgetupft, in ein Röhrchen gegeben, mit 5 ml frisch angesetzter Kollagenaselösung (Kollagenase (Cl. histolyticum) 0,74 U/mg, Serva, 2 mg/ml in HBBS/Wasser 1:9, pH 7,4) versetzt und unter Schütteln bei 37°C 18 Minuten inkubiert. Anschließend wird mit 1000 rpm 1 Minute zentrifugiert. Der Überstand wird verworfen. In einem zweiten Verdauungsschritt wird mit 5 ml Kollagenaselösung (1mg/ml) 4 Minuten inkubiert, geschüttelt und unverdautes Gewebe sedimentiert. Der Überstand wird dekantiert und der ganze Vorgang vier- bis fünfmal wiederholt. Der Überstand wird nun 1 Minute bei 1000 rpm zentrifugiert und die Kollagenaselösung verworfen. Das verbleibende Pellet wird mit eiskaltem HBBS aufgeschüttelt und ca. 10 Minuten auf Eis sedimentiert. Dieser Waschvorgang wird noch dreimal wiederholt. Aus den gewaschenen Pellets werden unter einer Stereolupe die schwach rosa angefärbten Inseln herausgepickt und in Kulturmedium (100ml RPMI 1640 (Gibco), 1 ml Glutamin, 1 ml Penicillin, 1 ml Cibrobay Antibiotikum (Bayer), 10 ml fötales Kälberserum, 2 ml Hepes-Puffer 1M) umgesetzt. Um eine möglichst reine Kultur zu erhalten, werden die Inseln zwei- bis dreimal gepickt und in frisches Kulturmedium umgesetzt.

**Stimulation der Inseln**

Die Inselzellen werden aus dem Kulturmedium zu je 10 Inseln in Eppendorfgefäße mit 200 ml Stimulationspuffer (NaCl 118 mM, $NaH_2PO_4$ 0,2 mM, $MgCl_2$ 0.565 mM, $CaCl_2$ 1,25 mM, Kcl 4,7 mM, Hepes 10 mM, BSA 1%, Glukose 3,3 mM; pH 7,4) verteilt und für eine Stunde bei 37°C in den Brutschrank gestellt. Anschließend werden die zu testenden Peptide zugegeben und mit Stimulationspuffer auf 500 ml aufgefüllt und eine Stunde bei 37°C inkubiert. Die Inseln werden bei 1000 rpm 1 Minute lang abzentrifugiert. Im Überstand wird die Menge an C-Peptid mit dem Insulin-RIA (DPC Biermann, Nauheim) gemessen. Jede Testsubstanz wurde vierfach bestimmt.

**Aktivität der Exendin-Analoga**

Einige Exendin-Analoga wurden wie oben beschriebenen an isolierten Inseln Zellen auf insulinsekretorische Aktivität getestet. Die Daten sind beispielhaft in folgender Tabelle:

**Insulinfreisetzung aus isolierten Inseln nach 1 Stunde [mIU/h/10 Inseln) in Anwesenheit von 10 mM Glukose:**

**Tabelle 6**

| | Kontrolle | GLP1-(7-36)-NH$_2$ | Seq.ID 84 |
|---|---|---|---|
| 10 mM Glukose | 30,21 | | |
| $10^{-7}$ (10mM Glukose) | | 53,52 | 48,94 |
| $10^{-8}$ (10mM Glukose) | | 42,78 | 41,72 |
| $10^{-9}$ (10mM Glukose) | | 29,99 | 38,76 |
| $10^{-10}$ (10mM Glukose) | | | 35,05 |

**Messung der Erhöhung der cytosolischen Calciumkonzentration in B-Zellen des endokrinen Pankreas (klonale B-Zellinie INS-1)**

**Zucht von INS-1-Zellen (Asfari, M., 1992):**

INS-I-Zetten werden in RPMI 1640 Medium mit 10% FKS, 10 mM HEPES-Puffer (pH, 7,4), 2 mM L-Glutamin, 100 i.U. Penicillin/ml, 100 μg Streptomycin/ml, 1 mM Pyruvat (Natriumsalz) und 50 μM 2-Mercaptoethanol kultiviert, bei 37° C, in einer Atmosphäre von 95 % Luft und 5 % $CO_2$. Nach 6 bis 8 Tagen Wachstum auf Kunststoff-Zellkulturplatten werden die subkonfluenten Zellen nach einmaligem Spülen mit PBS (phosphate-buffered saline) durch vierminütige Inkubation bei 37° C mit 0,025 % Trypsin und 0,27 mM EDTA in isoosmotischer Salzlösung von der Unterlage abgelöst.

**Präparation der Zellen für Calciummessungen:**

Die abgelösten Zellen werden in Spinnermedium (Kulturmedium wie oben, jedoch mit 5% FKS sowie 25 mM HEPES) resuspendiert und bei 37° C zweieinhalb Stunden in einer Spinnerflasche mit Rührstab inkubiert. Danach Entfernung des Mediums durch Zentrifugation und Resuspension der Zellen in Spinnermedium. Dann für 30 Minuten Inkubation bei 37° C mit 2 μM Fura-2/Acetoxymethylester, unter denselben Bedingungen wie zuvor. Die Fura-Beladung der Zellen wird durch einmaliges Waschen der Zellen in Spinnermedium (Raumtemperatur) beendet. Danach werden die Zellen in Spinnermedium mit Raumtemperatur resuspendiert (2 x $10^7$ Zellen/ml). Aus dieser Suspension werden die Zellen für Calciummessungen entnommen.

**Messungen der cytosolischen Calciumkonzentarion:**

Die Messungen erfolgen bei 37° C in einem modifizierten Krebs-Ringer Puffer (KRBH) mit 136 mM NaCl, 4,8 mM KCl, 2 mM CaCl$_2$, 1,2 mM MgSO$_4$, 1,2 mM KH$_2$PO$_4$, 5 mM NaHCO$_3$, 10 mM Glukose, 250 $\mu$M Sulfinpyrazon (zur Hemmung von Fura-2 Efflux in das Medium) und 25 mM HEPES-Puffer (mit NaOH auf pH 7,4). Die Zellkonzentration beträgt 1-2 x 10$^6$/m1. Die Messungen werden in einer mittels Rührstab gerührten Küvette in einem Spektralfluorimeter bei 37° C durchgeführt, mit 1,5 ml Zellsuspension. Exzitationswellenlänge ist 340 nm, Emissionswellenlänge 505 nm. Am Ende der Messungen werden 50 $\mu$M MnCl$_2$ und darauf 100 $\mu$M DTPA (Dieethylentriaminpentaacetat) zugegeben, um durch eine vorübergehende Löschung ("Quenching") der Fluoreszenz von extrazellulärem Fura den Anteil des extrazellulären Fluoreszenzindikators an der gemessenen Fluoreszenz bestimmen zu können. Nach der Zugabe von DTPA folgt die Überführung des gesamten Furas zunächst in einen calciumgesättigten und dann in einen calciumfreien Zustand, zur Ermittlung der Eichwerte F$_{max}$ (calciumgesättigt) und F$_{min}$ (calciumfrei) für die jeweilige Messung. Dazu werden die Zellen durch Zugabe von 0.1% Triton X-100 lysiert. Durch den Kontakt mit der hohen extrazellulären Calciumkonzentration wird der Farbstoff mit Calcium gesättigt. Danach werden 5 mM EGTA (Ethylenbis(oxyethylennitrilo)-tetraacetat) und 20 mM Tris-Lösung zugegeben, um den Farbstoff vollständig in die calciumfreie Form zu überführen.

Die Berechnung der cytosolischen Calciumionenkonzentration erfolgt nach dem von R. Tsien und Mitarbeitern eingeführten Algorhythmus (Grynkiewicz, G., 1985):

$$[Ca^{2+}]_{cyt} = ((F - F_{min})/(F_{max} - F)) \times K_D$$

(F: Fluoreszenz des jeweiligen Meßpunkts;

K$_D$: Dissoziationskonstante des Calciumkomplexes des Fura-2, 225 nM (Grynkiewicz, G., 1985))

(Vor dieser Berechnung wird eine Kompensation für die Anwesenheit von extrazellulärem Fura durchgeführt. Dazu wird zunächst der durch Manganzugabe ermittelte Fluoreszenzbetrag (extrazelluläres Fura) von den Fluoreszenzwerten der Meßpunkte subtrahiert. Dann wird F$_{max}$ durch die Subtraktion desselben Betrags korrigiert. Schließlich wurde der Korrekturbetrag für F$_{min}$ ermittelt. Dazu wird der durch Manganzugabe bestimmte Fluoreszenzbetrag durch den Wert 2,24 dividiert. Der Wert 2,24 war als geräteeigener Proportionalitätsfaktor zwischen der Fluoreszenz von calciumgesättigtem und calciumfreiem Fura-2 bei einer Exzitationswellenlänge von 340 nm bestimmt worden (gemessen mit unverestertem, freiem Fura-2). Der so erhaltene Korrekturbetrag wurde von F$_{min}$ subtrahiert.)

Die untersuchten Peptide wurden aus tausendfach konzentrierten Lösungen (10$^{-5}$ M) in KRBH ohne CaCl$_2$ und Glukose zugegeben.

**Aktivität der Exendin-Analoga**

Einige Exendin-Analoga wurden im oben beschriebenen Calcium-Assay an INS-1 Zellen auf ihre biologische Aktivität getestet. Die Daten sind beispielhaft in Abbildung 1 als auch in Tabelle 7 gezeigt.

**Tabelle 7**

| SEQ. ID Nr. | Konzentration der Peptide 10$^{-8}$ M | $\Delta[Ca^{2+}]cyt \pm SD(n=4)$ |
|---|---|---|
| 6 | [Arg$^{30}$]-Exendin-(1-30)-NH$_2$ | 67 $\pm$ 8 nM |
| 3 | [Nle$^{14}$, Arg$^{30}$]-Exendin-(1-30)- NH$_2$ | 63 $\pm$ 8 nM |
| 8 | [Ala$^{21}$, Arg$^{30}$]-Exendin-(1-30)- NH$_2$ | 61 $\pm$ 11 nM |
| 9 | [Ala$^{28}$, Arg$^{30}$]-Exendin-(1-30)- NH$_2$ | 65 $\pm$ 15 nM |
| 10 | [Ala$^{21,28}$, Arg$^{30}$]-Exendin-(1-30)- NH$_2$ | 69 $\pm$ 30 nM |
|  | Kontrolle: GLP-1-(7-36)amide | 65 $\pm$ 10 nM |

**Kompetition mit GLP1-(7-36)-NH$_2$ an B-Zellen des endokrinen Pankreas (klonale B-Zellinie INS-1)**

**Zucht von INS-1-Zellen (Asfari, M., 1992)**

siehe Messung Calciumconcentration

**Kompetitionsversuche**

Die abgelösten Zellen werden in Krebs-Ringer-Puffer (25 mM Tris, 120 mM NaCl, 1,2 mM MgSO$_4$ 5 mM Kcl, 1 mM Na-EDTA, 15 mM CH$_3$COONa, eingestellt auf pH 7,4, versetzt mit 1 % HSA und 0,1 % Bacitracin) aufgenommen und suspendiert. Aus dieser Suspension werden jeweils 250 ml für einen Ansatz entnommen, mit 20 ml Tracer ($^{125}$I-GLP1-(7-36)-NH$_2$, 20 000 cpm) und 30 ml des zu untersuchenden Peptides in der entsprechenden Verdünnung versetzt. Anschließend wird 30 Minuten bei 37°C inkubiert, 4 Minuten bei 13 000 rpm zentrifugiert, dreimal mit Puffer gewaschen und die am Pellet gebundene Radioaktivität (γ-Counter) gemessen. Kompetitionskurven wurden durch Inkubation mit 10 verschiedenen Verdünnungen des zu testenden Peptides ($10^{-10}$ - $10^{-6}$ M in Krebs-Ringer-Puffer) erhalten.

**Rezeptoraffinität der Exendin-Analoga**

Die Daten sind beispielhaft in Tabelle 8 aufgeführt. GLP1-(7-36)-NH$_2$ diente als Standard.

**Tabelle 8**

| Seq. ID | Peptid | Kd$_{GLP1}$ ± SD [nM] | Kd ± SD [nM] | Kd/Kd$_{GLP1}$ |
|---|---|---|---|---|
| 69 | [Nle$^{14}$, Tyr$^{30}$]-Ex3-(1-30)-NH$_2$ | 1,04±0,05 | 0,56 ± 0,08 | 0,5 |

**Literatur**

Albericio, F. and Barany, G. (1987) *Int. J. Peptide Protein Res.* **30,** 206-216.

Asfari, M., Janjic, D., Meda, P., Li, G., Halban, P. A. and Wollheim, C. B. (1992) *Endocrinology* **130,** 167-178.

Barlos, K., Gatos, D., Kapolos, S., Paphotiu, G., Schafer, W., and Wengqing, Y. (1989) *Tetrahedron Lett.* **30,** 3947-3950.

Booth, and Kenny, (1975) *Biochem. J.* **142,** 575-581.

Eng, J., Andrews, P. C., Kleinman, W. A., Singh, L., and Raufman, J.-P. (1990) *J. Biol. Chem.* **265**, 20259-20262.

Eng, J., Andrews, P. C., Kleinman, W. A., Singh, L., Singh, G., and Raufman, J.-P. (1992) *J. Biol. Chem.* **267,** 7402-7405.

Fehmann, H.C., Göke, R., Göke, B., Bachle, R., Wagner, B. and Arnold, R. (1991) *Biochim. Biophys Acta* **1091,** 356-63.

Göke, R., Wagner, B., Fehmann, H. C. and Göke, B. (1993a) *Res Exp. Med Berl.* **193,** 97-103

Göke, R., Fehman, H. C., Linn, T., Schmidt, H., Eng, J. and Göke, B. (1993b) *J. Biol. Chem.* **268,** 19650-19655.

Grynkiewicz, G., Poenie, M. and Tsien, R. Y. *(1985) J Biol Chem* **260**, 3440-3450.

Gutniak, M., Orskov, C., Holst, J. J., Ahren, B. and Efendic, S. (1992) *N. Engl. J. Med.* **326,** 1316-1322.

Komatsu, R., Matsuyama, T., Namba, M., Watanabe, N., Itoh, H., Kono, N.and Tarui, S. (1989) *Diabetes* **38**, 902-905.

Kreymann, B., Williams, G., Ghatei, M. A. and Bloom, S. R. (1987) *Lancet* 2(8571), 1300-1304.

Nathan, D.M., Schreiber, E., Fogel, H., Mojsov, S. and Habener, J. F. (1992) *Diabetes Care* **15**, 270-276.

Nauck, M. A., Kleine, N. Orskov, C., Holst, J. J., Willms, B. and Creutzfeld, W. (1993a) *Diabetologia* **36**, 741-744.

Nauck, M. A., Heimesaat, M. M., Orskov, C., Holst, J. J., Ebert, R. and Creutzfeld, W. (1993b) *J. Clin. Invest.* **91**, 301-307.

Raufman, J. P., Singh, L., Singh, G. and Eng, J.. (1992) *J. Biol. Chem.* **267**, 21432-21437.

Rink, H. (1987) *Tetrahedron Lett.* **28**, 3787-3790.

Thorens, B., Porret, A., Buehler, L., Deng, S.P., Morel, P. and Widman, C. (1993) *Diabetes* **42**, 1678-1682.

Wang, S. S. (1973) *J. Am. Chem. Soc.* **95,** 1328-1333.

SEQUENZPROTOKOLL

(1) ALLGEMEINE ANGABEN:

(i) ANMELDER:

(A) NAME: BOEHRINGER MANNHEIM GMBH

(B) STRASSE: Sandhofer Str. 116
(C) ORT: Mannheim
(E) LAND: Deutschland
(F) POSTLEITZAHL: D-68305
(G) TELEFON: 0621/759-3197
(H) TELEFAX: 0621/759-4457

(ii) BEZEICHNUNG DER ERFINDUNG: Exendin Analoga, Verfahren zu deren Herstellung und diese enthaltende Arzneimittel
(iii) ANZAHL DER SEQUENZEN: 118
(iv) COMPUTER-LESBARE FASSUNG:

(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30B (EPA)

(2) ANGABEN ZU SEQ ID NO: 1:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:30
(D) SONSTIGE ANGABEN:/product= "Xaa bedeutet alle Aminosaeuren ausser Gly"

(xi) SEQUENZBESCHREIBUNG: SEQ ID NO: 1:

```
His Ser Asp Gly Thr Phe Thr Ser Asp Leu Ser Lys Gln Met Glu Glu
1               5                   10                  15

Glu Ala Val Arg Leu Phe Ile Glu Trp Leu Lys Asn Gly Xaa
            20                  25                  30
```

(2) ANGABEN ZU SEQ ID NO: 2:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:30
(D) SONSTIGE ANGABEN:/product= "Xaa bedeutet alle Aminosaeuren ausser Gly"

(xi) SEQUENZBESCHREIBUNG: SEQ ID NO: 2:

```
His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Xaa
        20                  25                  30
```

(2) ANGABEN ZU SEQ ID NO: 3:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 3:

```
His Gly Glu Gly Thr Phe Thr Ser Asp Leu Ser Lys Gln Xaa Glu Glu
1               5                   10                  15

Glu Ala Val Arg Leu Phe Ile Glu Trp Leu Lys Asn Gly Arg
        20                  25                  30
```

(2) ANGABEN ZU SEQ ID NO: 4:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid

(ix) MERKMAL:

    (A) NAME/SCHLÜSSEL: Peptide
    (B) LAGE:14
    (D) SONSTIGE ANGABEN:/product= "Xaa bedeutet Nle"

(xi) SEQUENZBESCHREIBUNG: SEQ ID NO: 4:

```
His Gly Glu Gly Thr Phe Thr Ser Asp Leu Ser Lys Gln Xaa Glu Glu
1               5                   10                  15

Glu Ala Val Arg Leu Phe Ile Glu Trp Leu Lys Asn Gly Tyr
            20                  25                  30
```

(2) ANGABEN ZU SEQ ID NO: 5:

    (i) SEQUENZKENNZEICHEN:

        (A) LÄNGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (ix) MERKMAL:

        (A) NAME/SCHLUSSEL: Peptide
        (B) LAGE:14
        (D) SONSTIGE ANGABEN:/product= "Xaa bedeutet Nle"

    (xi) SEQUENZBESCHREIHUNG: SEQ ID NO: 5:

```
His Ser Asp Gly Thr Phe Thr Ser Asp Leu Ser Lys Gln Xaa Glu Glu
1               5                   10                  15

Glu Ala Val Arg Leu Phe Ile Glu Trp Leu Lys Asn Gly Arg
            20                  25                  30
```

(2) ANGABEN ZU SEQ ID NO: 6:

    (i) SEQUENZKENNZEICHEN:

        (A) LANGE: 30 Aminosäuren
        (B) ART: Aminosäure
        (C) STRANGFORM: Einzeistrang
        (D) TOPOLOGIE: linear

(ii) ART DES MOLEKULS: Peptid
(xi) SEQUENZBESCHREIBUNG: SEQ ID NO: 6:

```
His Gly Glu Gly Thr Phe Thr Ser Asp Leu Ser Lys Gln Met Glu Glu
1                   5                   10                  15

Glu Ala Val Arg Leu Phe Ile Glu Trp Leu Lys Asn Gly Arg
            20                  25                  30
```

(2) ANGABEN ZU SEQ ID NO: 7:

(i) SEQUENZKENNZEICHEN:

(A) LÄNGE: 29 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:13
(D) SONSTIGE ANGABEN:/product= "Xaa bedeutet Nle"

(xi) SEQUENZBESCHREIBUNG: SEQ ID NO: 7:

```
Gly Glu Gly Thr Phe Thr Ser Asp Leu Ser Lys Gln Xaa Glu Glu Glu
1                   5                   10                  15

Ala Val Arg Leu Phe Ile Glu Trp Leu Lys Asn Gly Arg
            20                  25
```

(2) ANGABEN ZU SEQ ID NO: 8:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(xi) SEQUENZBESCHREIBUNG: SEQ ID NO: 8:

His Gly Glu Gly Thr Phe Thr Ser Asp Leu Ser Lys Gln Met Glu Glu
1 5 10 15

Glu Ala Val Arg Ala Phe Ile Glu Trp Leu Lys Asn Gly Arg
20 25 30

(2) ANGABEN ZU SEQ ID NO: 9:

    (i) SEQUENZKENNZEICHEN:

        (A) LÄNGE: 30 Aminosäuren
        (B) ART: Amingsäure
        (C) STRANGFORM: Einzelstrang
        (D) TOPOLOGIE: linear

    (ii) ART DES MOLEKÜLS: Peptid
    (xi) SEQUENZBESCHREIBUNG: SEQ ID NO: 9:

His Gly Glu Gly Thr Phe Thr Ser Asp Leu Ser Lys Gln Met Glu Glu
1 5 10 15

Glu Ala Val Arg Leu Phe Ile Glu Trp Leu Lys Ala Gly Arg
20 25 30

(2) ANGABEN ZU SEQ ID NO: 10:

    (i) SEQUENZKENNZEICHEN:

        (A) LANGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (xi) SEQUENZBESCHREIBUNG: SEQ ID NO: 10:

His Gly Glu Gly Thr Phe Thr Ser Asp Leu Ser Lys Gln Met Glu Glu
1                   5                   10                  15

Glu Ala Val Arg Ala Phe Ile Glu Trp Leu Lys Ala Gly Arg
                20                  25                  30

(2) ANGABEN ZU SEQ ID NO: 11:

   (i) SEQUENZKENNZEICHEN:

      (A) LÄNGE: 30 Aminosäuren
      (B) ART: Aminosäure
      (C) STRANGFORM: Einzelstrang
      (D) TOPOLOGIE: linear

   (ii) ART DES MOLEKÜLS: Peptid
   (xi) SEQUENZBESCHREIBUNG: SEQ ID NO: 11:

His Gly Glu Gly Thr Phe Thr Ser Asp Leu Ser Lys Gln Ala Glu Glu
1                   5                   10                  15

Glu Ala Val Arg Leu Phe Ile Glu Trp Leu Lys Asn Gly Arg
                20                  25                  30

(2) ANGABEN ZU SEQ ID NO: 12:

   (i) SEQUENZKENNZEICHEN:

      (A) LÄNGE: 30 Aminosäuren
      (B) ART: Aminosäure
      (C) STRANGFORM: Einzelstrang
      (D) TOPOLOGIE: linear

   (ii) ART DES MOLEKÜLS: Peptid
   (xi) SEQUENZBESCHREIBUNG: SEQ ID NO: 12:

His Gly Glu Gly Thr Phe Thr Ser Asp Leu Ser Lys Gln Ile Glu Glu
1                   5                   10                  15

Glu Ala Val Arg Leu Phe Ile Glu Trp Leu Lys Asn Gly Arg

20          25          .          30

(2) ANGABEN ZU SEQ ID NO: 13:

(i) SEQUENZKENNZEICHEN:

(A) LANGE: 27 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 13:

His Gly Glu Gly Thr Phe Thr Ser Asp Leu Ser Lys Gln Xaa Glu Glu
1                   5                   10                  15

Glu Ala Val Arg Leu Phe Ile Glu Trp Leu Lys
                20                  25

(2) ANGABEN ZU SEQ ID NO: 14:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 14:

```
His Ser Asp Gly Thr Phe Thr Ser Asp Leu Ser Lys Gln Ala Glu Glu
 1               5                10                15
```

```
Glu Ala Val Arg Leu Phe Ile Glu Trp Leu Lys Asn Ala Arg
         20                25                30
```

(2) ANGABEN ZU SEQ ID NO: 15:

    (i) SEQUENZKENNZEICHEN:

        (A) LÄNGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (ix) MERKMAL:

        (A) NAME/SCHLÜSSEL: Peptide
        (B) LAGE:14
        (D) SONSTIGE ANGABEN:/product= "Xaa bedeutet Nle"

    (xi) SEQUENZBESCHREIBUNG:

```
    Glu Ala Val Arg Lesp Gly Thr Phe Thr Ser Asp Leu Ser Lys Gln Ala
Glu Glu
 1               5                10                15
```

```
O: 3:
```

```
    (i) SEQUENZKENNZEIle Glu Trp Leu Ala Asn Gly Arg
         20                25                30
```

(2) ANGABEN ZU SEQ ID NO: 16:

    (i) SEQUENZKENNZEICHEN:

    (A) LÄNGE: 30 Aminosäuren
    (B) ART: Aminosäure
    (C) STRANGFORM: Einzelstrang
    (D) TOPOLOGIE: linear

    (ii) ART DES MOLEKÜLS: Peptid
    (ix) MERKMAL:

    (A) NAME/SCHLUSSEL: Peptide
    (B) LAGE:14

(D) SONSTIGE ANGABEN:/product= "Xaa bedeutet Nle"

(xi) SEQUENZBESCHREIBUNG: SEQ ID NO: 16:

His Ser Asp Gly Thr Phe Thr Ser Asp Leu Ser Lys Gln Ala Glu Glu

1                 5                 10                 15

Glu Ala Val Arg Leu Phe Ile Glu Trp Ala Lys Asn Gly Arg

             20                 25                 30

(2) ANGABEN ZU SEQ ID N (D) TOPOLOGIE: linear

(ii) ART DES MOLEKÜLS: Peptid
Aminosäuren

(B) ART: A) NAME/SCHLÜSSEL: Peptide
FORM: Einzelstrang
(D) TOPOLOGIE: linear

(ii) ART DES MOLEKULS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 17:

His Ala Glu Gly Thr Phe Thr Ser Asp Leu Ser Lys Gln Xaa Glu Glu

1                 5                 10                 15

Glu Ala Val Arg Leu Phe Ile Glu Trp Leu Lys Asn Gly Arg

             20                 25                 30

(2) ANGABEN ZU SEQ ID NO: 18:

(i) SEQUENZKENNZEICHEN:

(A) LÄNGE: 30 Aminosäuren
(B) ART: Aminosäure
(C)
(B) LAGE:14
(D) TOPOLOGIE: linear

(ii) ART DES MOLEKÜLS: Peptid
(ix) MERKMAL:

(A) NAME/SCHLUSSEL: Peptide
(B) LAGE:14
(D) SONSTIGE ANGABEN:/product= "Xaa bedeutet Nle"

(xi) SEQUENZBESCHREIBUNG: SEQ ID NO: 18:

```
His Cys Glu Gly Thr Phe Thr Ser Asp Leu Ser Lys Gln Xaa Glu Glu
1               5                   10                  15
```

```
Glu Ala Val Arg Leu Phe Ile Glu Trp Leu Lys Asn Gly Arg
                20                  25                  30
```

(2) ANGABEN ZU SEQ ID NO: 19:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 19:

```
His Asp Glu Gly Thr Phe Thr Ser Asp Leu Ser Lys Gln Xaa Glu Glu
1               5                   10                  15
```

```
Glu Ala Val Arg Leu Phe Ile Glu Trp Leu Lys Asn Gly Arg
                20                  25                  30
```

(2) ANGABEN ZU SEQ ID NO: 20:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 20:

```
His Glu Glu Gly Thr Phe Thr Ser Asp Leu Ser Lys Gln Xaa Glu Glu
1                   5                   10                  15


Glu Ala Val Arg Leu Phe Ile Glu Trp Leu Lys Asn Gly Arg
                20                  25                  30
```

(2) ANGABEN ZU SEQ ID NO: 21:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 21:

```
His Phe Glu Gly Thr Phe Thr Ser Asp Leu Ser Lys Gln Xaa Glu Glu
1                   5                   10                  15


Glu Ala Val Arg Leu Phe Ile Glu Trp Leu Lys Asn Gly Arg
                20                  25                  30
```

(2) ANGABEN ZU SEQ ID NO: 22:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

    (A) NAME/SCHLÜSSEL: Peptide
    (B) LAGE:14
    (D) SONSTIGE ANGABEN:/product= "Xaa bedeutet Nle"

(xi) SEQUENZBESCHREIBUNG: SEQ ID NO: 22:

```
His His Glu Gly Thr Phe Thr Ser Asp Leu Ser Lys Gln Xaa Glu Glu
1               5                   10                  15


Glu Ala Val Arg Leu Phe Ile Glu Trp Leu Lys Asn Gly Arg
            20                  25                  30
```

(2) ANGABEN ZU SEQ ID NO: 23:

    (i) SEQUENZKENNZEICHEN:

        (A) LÄNGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (ix) MERKMAL:

        (A) NAME/SCHLÜSSEL: Peptide
        (B) LAGE:14
        (D) SONSTIGE ANGABEN:/product= "Xaa bedeutet Nle"

    (xi) SEQUENZBESCHREIBUNG: SEQ ID NO: 23:

```
His Ile Glu Gly Thr Phe Thr Ser Asp Leu Ser Lys Gln Xaa Glu Glu
1               5                   10                  15


Glu Ala Val Arg Leu Phe Ile Glu Trp Leu Lys Asn Gly Arg
            20                  25                  30
```

(2) ANGABEN ZU SEQ ID NO: 24:

    (i) SEQUENZKENNZEICHEN:

        (A) LANGE: 30 Aminosäuren
        (B) ART: Aminosäure
        (C) STRANGFORM: Einzelstrang

(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 24:

```
His Lys Glu Gly Thr Phe Thr Ser Asp Leu Ser Lys Gln Xaa Glu Glu
1               5                   10                  15

Glu Ala Val Arg Leu Phe Ile Glu Trp Leu Lys Asn Gly Arg
        20                  25                  30
```

(2) ANGABEN ZU SEQ ID NO: 25:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 25:

```
His Leu Glu Gly Thr Phe Thr Ser Asp Leu Ser Lys Gln Xaa Glu Glu
1               5                   10                  15

Glu Ala Val Arg Leu Phe Ile Glu Trp Leu Lys Asn Gly Arg
        20                  25                  30
```

(2) ANGABEN ZU SEQ ID NO: 26:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 26:

```
His Met Glu Gly Thr Phe Thr Ser Asp Leu Ser Lys Gln Xaa Glu Glu
1               5                   10                  15
```

```
Glu Ala Val Arg Leu Phe Ile Glu Trp Leu Lys Asn Gly Arg
            20                  25                  30
```

(2) ANGABEN ZU SEQ ID NO: 27:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKULS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 27:

```
His Asn Glu Gly Thr Phe Thr Ser Asp Leu Ser Lys Gln Xaa Glu Glu
1               5                   10                  15
```

```
Glu Ala Val Arg Leu Phe Ile Glu Trp Leu Lys Asn Gly Arg
            20                  25                  30
```

(2) ANGABEN ZU SEQ ID NO: 28:

(i) SEQUENZKENNZEICHEN:

    (A) LANGE: 30 Aminosäuren
    (B) ART: Aminosäure
    (C) STRANGFORM: Einzelstrang
    (D) TOPOLOGIE: linear

(ii) ART DES MOLEKÜLS: Peptid
(ix) MERKMAL:

    (A) NAME/SCHLÜSSEL: Peptide
    (B) LAGE:14
    (D) SONSTIGE ANGABEN:/product= "Xaa bedeutet Nle"

(xi) SEQUENZBESCHREIBUNG: SEQ ID NO: 28:

```
His Pro Glu Gly Thr Phe Thr Ser Asp Leu Ser Lys Gln Xaa Glu Glu
 1               5                  10                  15

Glu Ala Val Arg Leu Phe Ile Glu Trp Leu Lys Asn Gly Arg
            20                  25                  30
```

(2) ANGABEN ZU SEQ ID NO: 29:

(i) SEQUENZKENNZEICHEN:

    (A) LÄNGE: 30 Aminosäuren
    (B) ART: Aminosäure
    (C) STRANGFORM: Einzelstrang
    (D) TOPOLOGIE: linear

(ii) ART DES MOLEKULS: Peptid

(ix) MERKMAL:

    (A) NAME/SCHLUSSEL: Peptide
    (B) LAGE:14
    (D) SONSTIGE ANGABEN:/product= "Xaa bedeutet Nle"

(xi) SEQUENZBESCHREIBUNG: SEQ ID NO: 29:

```
His Gln Glu Gly Thr Phe Thr Ser Asp Leu Ser Lys Gln Xaa Glu Glu
 1               5                  10                  15

Glu Ala Val Arg Leu Phe Ile Glu Trp Leu Lys Asn Gly Arg
            20                  25                  30
```

(2) ANGABEN ZU SEQ ID NO: 30:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 30:

```
His Arg Glu Gly Thr Phe Thr Ser Asp Leu Ser Lys Gln Xaa Glu Glu
1               5                   10                  15

Glu Ala Val Arg Leu Phe Ile Glu Trp Leu Lys Asn Gly Arg
            20                  25                  30
```

(2) ANGABEN ZU SEQ ID NO: 31:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 31:

```
His Ser Glu Gly Thr Phe Thr Ser Asp Leu Ser Lys Gln Xaa Glu Glu
1                 5                 10                15
```

```
Glu Ala Val Arg Leu Phe Ile Glu Trp Leu Lys Asn Gly Arg
            20                25                30
```

(2) ANGABEN ZU SEQ ID NO: 32:

    (i) SEQUENZKENNZEICHEN:

        (A) LÄNGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (ix) MERKMAL:

        (A) NAME/SCHLÜSSEL: Peptide
        (B) LAGE:14
        (D) SONSTIGE ANGABEN:/product= "Xaa bedeutet Nle"

    (xi) SEQUENZBESCHREIBUNG: SEQ ID NO: 32:

```
His Thr Glu Gly Thr Phe Thr Ser Asp Leu Ser Lys Gln Xaa Glu Glu
1                 5                 10                15
```

```
Glu Ala Val Arg Leu Phe Ile Glu Trp Leu Lys Asn Gly Arg
            20                25                30
```

(2) ANGABEN ZU SEQ ID NO: 33:

    (i) SEQUENZKENNZEICHEN:

        (A) LANGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (ix) MERKMAL:

        (A) NAME/SCHLÜSSEL: Peptide
        (B) LAGE:14
        (D) SONSTIGE ANGABEN:/product= "Xaa bedeutet Nle"

(xi) SEQUENZBESCHREIBUNG: SEQ ID NO: 33:

His Val Glu Gly Thr Phe Thr Ser Asp Leu Ser Lys Gln Xaa Glu Glu
1               5                   10                  15

Glu Ala Val Arg Leu Phe Ile Glu Trp Leu Lys Asn Gly Arg
        20                  25                  30

(2) ANGABEN ZU SEQ ID NO: 34:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKULS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 34:

His Trp Glu Gly Thr Phe Thr Ser Asp Leu Ser Lys Gln Xaa Glu Glu
1               5                   10                  15

Glu Ala Val Arg Leu Phe Ile Glu Trp Leu Lys Asn Gly Arg
        20                  25                  30

(2) ANGABEN ZU SEQ ID NO: 35:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid

(ix) MERKMAL:

  (A) NAME/SCHLÜSSEL: Peptide
  (B) LAGE:14
  (D) SONSTIGE ANGABEN:/product= "Xaa bedeutet Nle"

(xi) SEQUENZBESCHREIBUNG: SEQ ID NO: 35:

```
His Tyr Glu Gly Thr Phe Thr Ser Asp Leu Ser Lys Gln Xaa Glu Glu
1               5                   10                  15
```

```
Glu Ala Val Arg Leu Phe Ile Glu Trp Leu Lys Asn Gly Arg
        20                  25                  30
```

(2) ANGABEN ZU SEQ ID NO: 36:

  (i) SEQUENZKENNZEICHEN:

    (A) LÄNGE: 30 Aminosäuren
    (B) ART: Aminosäure
    (C) STRANGFORM: Einzelstrang
    (D) TOPOLOGIE: linear

  (ii) ART DES MOLEKÜLS: Peptid
  (xi) SEQUENZBESCHREIBUNG: SEQ ID NO: 36:

```
His Ala Asp Gly Thr Phe Thr Ser Asp Leu Ser Ser Tyr Met Glu Gly
1               5                   10                  15
```

```
Gln Ala Val Lys Glu Phe Ile Ala Trp Leu Val Lys Gly Arg
        20                  25                  30
```

(2) ANGABEN ZU SEQ ID NO: 37:

  (i) SEQUENZKENNZEICHEN:

    (A) LÄNGE: 30 Aminosäuren
    (B) ART: Aminosäure
    (C) STRANGFORM: Einzelstrang
    (D) TOPOLOGIE: linear

  (ii) ART DES MOLEKÜLS: Peptid
  (xi) SEQUENZBESCHREIBUNG: SEQ ID NO: 37:

```
His Ser Asp Gly Thr Phe Thr Ser Asp Leu Ser Lys Gln Ala Glu Glu
1               5                   10                  15

Glu Ala Val Arg Leu Phe Ile Glu Ala Leu Lys Asn Gly Arg
            20                  25                  30
```

(2) ANGABEN ZU SEQ ID NO: 38:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKULS: Peptid
(xi) SEQUENZBESCHREIBUNG: SEQ ID NO: 38:

```
His Ser Glu Gly Thr Phe Thr Ser Asp Leu Ser Lys Gln Ala Glu Glu
1               5                   10                  15

                .

Glu Ala Val Arg Leu Phe Ile Glu Trp Leu Lys Asn Gly Arg
            20                  25                  30
```

(2) ANGABEN ZU SEQ ID NO: 39:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKULS: Peptid
(xi) SEQUENZBESCHREIBUNG: SEQ ID NO: 39:

```
Ala Ser Asp Gly Thr Phe Thr Ser Asp Leu Ser Lys Gln Val Glu Glu
1               5                   10                  15
```

```
Glu Ala Val Arg Leu Phe Ile Glu Trp Leu Lys Asn Gly Arg
            20                  25                  30
```

(2) ANGABEN ZU SEQ ID NO: 40:

    (i) SEQUENZKENNZEICHEN:

        (A) LANGE: 30 Aminosäuren
        (B) ART: Aminosäure
        (C) STRANGFORM: Einzelatrang
        (D) TOPOLOGIE: linear

    (ii) ART DES MOLEKÜLS: Peptid
    (xi) SEQUENZBESCHREIBUNG: SEQ ID NO: 40:

```
His Gly Ala Gly Thr Phe Thr Ser Asp Leu Ser Lys Gln Ala Glu Glu
1               5                   10                  15
```

```
Glu Ala Val Arg Leu Phe Ile Glu Trp Leu Lys Asn Gly Arg
            20                  25                  30
```

(2) ANGABEN ZU SEQ ID NO: 41:

    (i) SEQUENZKENNZEICHEN:

        (A) LANGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (xi) SEQUENZBESCHREIBUNG: SEQ ID NO: 41:

His Gly Glu Gly Ala Phe Thr Ser Asp Leu Ser Lys Gln Ala Glu Glu
1               5                   10                  15

Glu Ala Val Arg Leu Phe Ile Glu Trp Leu Lys Asn Gly Arg
            20                  25                  30

(2) ANGABEN ZU SEQ ID NO: 42:

    (i) SEQUENZKENNZEICHEN:

        (A) LÄNGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (xi) SEQUENZBESCHREIBUNG: SEQ ID NO: 42:

His Gly Glu Gly Tyr Phe Thr Ser Asp Leu Ser Lys Gln Ala Glu Glu
1               5                   10                  15

Glu Ala Val Arg Leu Phe Ile Glu Trp Leu Lys Asn Gly Arg
            20                  25                  30

(2) ANGABEN ZU SEQ ID NO: 43:

    (i) SEQUENZKENNZEICHEN:

        (A) LÄNGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (xi) SEQUENZBESCHREIBUNG: SEQ ID NO: 43:

His Gly Glu Gly Thr Tyr Thr Ser Asp Leu Ser Lys Gln Ala Glu Glu
1               5                   10                  15

Glu Ala Val Arg Leu Phe Ile Glu Trp Leu Lys Asn Gly Arg
            20                  25                  30

(2) ANGABEN ZU SEQ ID NO: 44:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Genom-DNA
(xi) SEQUENZBESCHREIBUNG: SEQ ID NO: 44:

His Gly Glu Gly Thr Ile Thr Ser Asp Leu Ser Lys Gln Ala Glu Glu
1               5                   10                  15

Glu Ala Val Arg Leu Phe Ile Glu Trp Leu Lys Asn Gly Arg
            20                  25                  30

(2) ANGABEN ZU SEQ ID NO: 45:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(xi) SEQUENZBESCHREIBUNG: SEQ ID NO: 45:

```
His Gly Glu Gly Thr Phe Ser Ser Asp Leu Ser Lys Gln Ala Glu Glu
1               5                   10                  15
```

```
Glu Ala Val Arg Leu Phe Ile Glu Trp Leu Lys Asn Gly Arg
        20                  25                  30
```

(2) ANGABEN ZU SEQ ID NO: 46:

    (i) SEQUENZKENNZEICHEN:

        (A) LANGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (xi) SEQUENZBESCHREIBUNG: SEQ ID NO: 46:

```
His Gly Glu Gly Thr Phe Tyr Ser Asp Leu Ser Lys Gln Ala Glu Glu
1               5                   10                  15
```

```
Glu Ala Val Arg Leu Phe Ile Glu Trp Leu Lys Asn Gly Arg
        20                  25                  30
```

(2) ANGABEN ZU SEQ ID NO: 47:

    (i) SEQUENZKENNZEICHEN:

        (A) LANGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (xi) SEQUENZBESCHREIBUNG: SEQ ID NO: 47:

```
His Gly Glu Gly Thr Phe Thr Thr Asp Leu Ser Lys Gln Ala Glu Glu
 1               5               10              15
```

```
Glu Ala Val Arg Leu Phe Ile Glu Trp Leu Lys Asn Gly Arg
          20              25              30
```

.

(2) ANGABEN ZU SEQ ID NO: 48:

(i) SEQUENZKENNZEICHEN:

(A) LANGE: 30 Aminosäuren
(B) ART: Aminosäure
(c) STRANGFORM: Einzelstrang
(D) TOPOLOGIE: linear

(ii) ART DES MOLEKÜLS: Peptid
(xi) SEQUENZBESCHREIBUNG: SEQ ID NO: 48:

```
His Gly Glu Gly Thr Phe Thr Tyr Asp Leu Ser Lys Gln Ala Glu Glu
 1               5               10              15
```

```
Glu Ala Val Arg Leu Phe Ile Glu Trp Leu Lys Asn Gly Arg
          20              25              30
```

(2) ANGABEN ZU SEQ ID NO: 49:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(xi) SEQUENZBESCHREIBUNG: SEQ ID NO: 49:

His Gly Glu Gly Thr Phe Thr Ser Glu Leu Ser Lys Gln Ala Glu Glu
1                   5                   10                  15

Glu Ala Val Arg Leu Phe Ile Glu Trp Leu Lys Asn Gly Arg
        20                  25                  30

(2) ANGABEN ZU SEQ ID NO: 50:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(xi) SEQUENZBESCHREIBUNG: SEQ ID NO: 50:

His Gly Glu Gly Thr Phe Thr Ser Asp Ala Ser Lys Gln Ala Glu Glu
1                   5                   10                  15

Glu Ala Val Arg Leu Phe Ile Glu Trp Leu Lys Asn Gly Arg
        20                  25                  30

(2) ANGABEN ZU SEQ ID NO: 51:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKULS: Peptid
(xi) SEQUENZBESCHREIBUNG: SEQ ID NO: 51:

His Gly Glu Gly Thr Phe Thr Ser Asp Leu Ala Lys Gln Ala Glu Glu
1               5                   10                  15

Glu Ala Val Arg Leu Phe Ile Glu Trp Leu Lys Asn Gly Arg
                20                  25                  30

(2) ANGABEN ZU SEQ ID NO: 52:

    (i) SEQUENZKENNZEICHEN:

        (A) LANGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (xi) SEQUENZBESCHREIBUNG: SEQ ID NO: 52:

His Gly Glu Gly Thr Phe Thr Ser Asp Leu Ser Ala Gln Ala Glu Glu

1               5                   10                  15

Glu Ala Val Arg Leu Phe Ile Glu Trp Leu Lys Asn Gly Arg
                20                  25                  30

(2) ANGABEN ZU SEQ ID NO: 53:

    (i) SEQUENZKENNZEICHEN:

        (A) LANGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (xi) SEQUENZBESCHREIBUNG: SEQ ID NO: 53:

```
His Gly Glu Gly Thr Phe Thr Ser Asp Leu Ser Lys Ala Ala Glu Glu
 1               5                10                 15
```

```
Glu Ala Val Arg Leu Phe Ile Glu Trp Leu Lys Asn Gly Arg
          20                25                30
```

(2) ANGABEN ZU SEQ ID NO: 54:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(xi) SEQUENZBESCHREIBUNG: SEQ ID NO: 54:

```
His Gly Glu Gly Thr Phe Thr Ser Asp Leu Ser Lys Gln Ala Ala Glu
 1               5                10                 15
```

```
Glu Ala Val Arg Leu Phe Ile Glu Trp Leu Lys Asn Gly Arg
          20                25                30
```

(2) ANGABEN ZU SEQ ID NO: 55:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(xi) SEQUENZBESCHREIBUNG: SEQ ID NO: 55:

```
Gly Ser Asp Gly Ser Phe Thr Ser Asp Leu Ser Lys Gln Ala Glu Ala
 1               5                10                 15
```

```
        Glu Ala Val Arg Leu Phe Ile Glu Trp Leu Lys Asn Gly Arg
                    20              25                  30
```

(2) ANGABEN ZU SEQ ID NO: 56:

    (i) SEQUENZKENNZEICHEN:

        (A) LANGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (xi) SEQUENZBESCHREIBUNG: SEQ ID NO: 56:

```
        Lys Gly Glu Gly Thr Phe Thr Ser Asp Leu Ser Lys Gln Ala Glu Glu
        1               5                   10                  15


        Ala Ala Val Arg Leu Phe Ile Glu Trp Leu Lys Asn Gly Arg
                    20              25                  30
```

(2) ANGABEN ZU SEQ ID NO: 57:

    (i) SEQUENZKENNZEICHEN:

        (A) LANGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (xi) SEQUENZBESCHREIBUNG: SEQ ID NO: 57:

```
        His Gly Glu Gly Thr Phe Thr Ser Asp Leu Ser Lys Gln Ala Glu Glu
        1               5                   10                  15


        Glu Leu Val Arg Leu Phe Ile Glu Trp Leu Lys Asn Gly Arg
                    20              25                  30
```

(2) ANGABEN ZU SEQ ID NO: 58:

    (i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(xi) SEQUENZBESCHREIBUNG: SEQ ID NO: 58:


His Gly Glu Gly Thr Phe Thr Ser Asp Leu Ser Lys Gln Ala Glu Glu
1                   5                   10                  15


Glu Ala Ile Arg Leu Phe Ile Glu Trp Leu Lys Asn Gly Arg
                    20                  25                  30


(2) ANGABEN ZU SEQ ID NO: 59:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(xi) SEQUENZBESCHREIBUNG: SEQ ID NO: 59:


His Gly Glu Gly Thr Phe Thr Ser Asp Leu Ser Lys Gln Ala Glu Glu
1                   5                   10                  15


Glu Ala Val Ala Leu Phe Ile Glu Trp Leu Lys Asn Gly Arg
                    20                  25                  30


(2) ANGABEN ZU SEQ ID NO: 60:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(xi) SEQUENZBESCHREIBUNG: SEQ ID NO: 60:

```
His Ser Asp Gly Thr Phe Thr Ser Asp Leu Ser Lys Gln Ala Glu Glu
 1               5                  10                  15

Glu Ala Val Arg Leu Tyr Ile Glu Trp Leu Lys Asn Gly Arg
         20                  25                  30
```

(2) ANGABEN ZU SEQ ID NO: 61:

    (i) SEQUENZKENNZEICHEN:

        (A) LANGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (xi) SEQUENZBESCHREIBUNG: SEQ ID NO: 61:

```
His Gly Glu Gly Thr Phe Thr Ser Asp Leu Ser Lys Gln Ala Glu Glu
 1               5                  10                  15

Glu Ala Val Arg Leu Phe Val Glu Trp Leu Lys Asn Gly Arg
         20                  25                  30
```

(2) ANGABEN ZU SEQ ID NO: 62:

    (i) SEQUENZKENNZEICHEN:

        (A) LANGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (xi) SEQUENZBESCHREIBUNG: SEQ ID NO: 62:

```

His Gly Glu Gly Thr Phe Thr Ser Asp Leu Ser Lys Gln Ala Glu Glu
1 5 10 15

Glu Ala Val Arg Leu Phe Ile Leu Trp Leu Lys Asn Gly Arg
20 25 30

(2) ANGABEN ZU SEQ ID NO: 63:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(xi) SEQUENZBESCHREIBUNG: SEQ ID NO: 63:

His Gly Glu Gly Thr Phe Thr Ser Asp Leu Ser Lys Gln Ala Glu Glu
1 5 10 15

Glu Ala Val Arg Leu Phe Ile Glu Ala Leu Lys Asn Gly Arg
20 25 30

(2) ANGABEN ZU SEQ ID NO: 64:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(xi) SEQUENZBESCHREIBUNG: SEQ ID NO: 64:

```
His Gly Glu Gly Thr Phe Thr Ser Asp Leu Ser Lys Gln Ala Glu Glu
 1               5                  10                  15
```

```
Glu Ala Val Arg Leu Phe Ile Glu Trp Ala Lys Asn Gly Arg
        20                  25                  30
```

(2) ANGABEN ZU SEQ ID NO: 65:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(xi) SEQUENZBESCHREIBUNG: SEQ ID NO: 65:

```
His Gly Glu Gly Thr Phe Thr Ser Asp Leu Ser Lys Gln Ala Glu Glu
 1               5                  10                  15
```

```
Glu Ala Val Arg Leu Phe Ile Glu Trp Leu Ala Asn Gly Arg
        20                  25                  30
```

(2) ANGABEN ZU SEQ ID NO: 66:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(xi) SEQUENZBESCHREIBUNG: SEQ ID NO: 66:

```
His Gly Glu Gly Thr Phe Thr Ser Asp Leu Ser Lys Gln Ala Glu Glu
1               5                   10                  15
```

```
Glu Ala Val Arg Leu Phe Ile Glu Trp Leu Lys Asn Ala Arg
            20                  25                  30
```

(2) ANGABEN ZU SEQ ID NO: 67:

    (i) SEQUENZKENNZEICHEN:

      (A) LÄNGE: 30 Aminosäuren
      (B) ART: Aminosäure
      (C) STRANGFORM: Einzelstrang
      (D) TOPOLOGIE: linear

    (ii) ART DES MOLEKÜLS: Peptid
    (xi) SEQUENZBESCHREIBUNG: SEQ ID NO: 67:

```
His Gly Glu Gly Thr Phe Thr Ser Asp Leu Ser Lys Gln Ala Glu Glu
1               5                   10                  15
```

```
Glu Ala Val Arg Leu Phe Ile Glu Trp Leu Lys Asn Gly Arg
            20                  25                  30
```

(2) ANGABEN ZU SEQ ID NO: 68:

    (i) SEQUENZKENNZEICHEN:

      (A) LÄNGE: 30 Aminosäuren
      (B) ART: Aminosäure
      (C) STRANGFORM: Einzelstrang
      (D) TOPOLOGIE: linear

    (ii) ART DES MOLEKÜLS: Peptid
    (xi) SEQUENZBESCHREIBUNG: SEQ ID NO: 68:

```
His Ser Asp Gly Thr Phe Thr Ser Asp Leu Ser Lys Gln Met Glu Glu
1               5                   10                  15
```

```
Glu Ala Val Arg Leu Phe Ile Glu Trp Leu Lys Asn Gly Arg
        20                  25                  30
```

(2) ANGABEN ZU SEQ ID NO: 69:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product = "Xaa bedeutet Nle"

(xi) SEQUENZBESCHREIBUNG: SEQ ID NO: 69:

```
His Ser Asp Gly Thr Phe Thr Ser Asp Leu Ser Lys Gln Xaa Glu Glu
1               5                   10                  15
```

```
Glu Ala Val Arg Leu Phe Ile Glu Trp Leu Lys Asn Gly Tyr
        20                  25                  30
```

(2) ANGABEN ZU SEQ ID NO: 70:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLUSSEL: Peptide
(B) LAGE:14
(D) SONSTIGE ANGABEN:/product= "Xaa bedeutet Nle"

(xi) SEQUENZBESCHREIBUNG: SEQ ID NO: 70:

His Ser Asp Gly Thr Phe Thr Ser Asp Leu Ser Lys Gln Xaa Glu Glu

Glu Ala Val Arg Leu Phe Ile Glu Trp Leu Lys Asn Gly Arg

(2) ANGABEN ZU SEQ ID NO: 71:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(xi) SEQUENZBESCHREIBUNG: SEQ ID NO: 71:

His Ser Asp Gly Thr Phe Thr Ser Asp Leu Ser Lys Gln Ala Glu Glu

Glu Ala Val Arg Leu Phe Ile Glu Trp Leu Lys Asn Gly Arg

(2) ANGABEN ZU SEQ ID NO: 72:

(i) SEQUENZKENNZEICHEN:

(A) LÄNGE: 29 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:13
(D) SONSTIGE ANGABEN:/product= "Xaa bedeutet Nle"

(xi) SEQUENZBESCHREIBUNG: SEQ ID NO: 72:

```
Ser Asp Gly Thr Phe Thr Ser Asp Leu Ser Lys Gln Xaa Glu Glu Glu
1                   5                   10                  15

Ala Val Arg Leu Phe Ile Glu Trp Leu Lys Asn Gly Arg
            20                  25
```

(2) ANGABEN ZU SEQ ID NO: 73:

    (i) SEQUENZKENNZEICHEN:

        (A) LANGE: 28 Aminosäuren
        (B) ART: Aminosäure
        (C) STRANGFORM: Einzelstrang
        (D) TOPOLOGIE: linear

    (ii) ART DES MOLEKÜLS: Peptid
    (ix) MERKMAL:

        (A) NAME/SCHLÜSSEL: Peptide
        (B) LAGE:12
        (D) SONSTIGE ANGABEN:/product= "Xaa bedeutet Nle"

    (xi) SEQUENZBESCHREIBUNG: SEQ ID NO: 73:

```
Asp Gly Thr Phe Thr Ser Asp Leu Ser Lys Gln Xaa Glu Glu Glu Ala
1                   5                   10                  15

Val Arg Leu Phe Ile Glu Trp Leu Lys Asn Gly Arg
            20                  25
```

(2) ANGABEN ZU SEQ ID NO: 74:

    (i) SEQUENZKENNZEICHEN:

        (A) LANGE: 29 Aminosäuren
        (B) ART: Aminosäure
        (C) STRANGFORM; Einzelstrang
        (D) TOPOLOGIE: linear

    (ii) ART DES MOLEKÜLS: Peptid
    (ix) MERKMAL:

        (A) NAME/SCHLÜSSEL: Peptide
        (B) LAGE:13
        (D) SONSTIGE ANGABEN:/product= "Xaa bedeutet Nle"

EP 0 915 910 B1

(xi) SEQUENZBESCHREIBUNG: SEQ ID NO: 74:

Ser Asp Gly Thr Phe Thr Ser Asp Leu Ser Lys Gln Xaa Glu Glu Glu

Ala Val Arg Leu Phe Ile Glu Trp Leu Lys Asn Gly Arg

(2) ANGABEN ZU SEQ ID NO: 75:

(i) SEQUENZKENNZEICHEN:

(A) LANGE: 28 Aminosäuren
(B) ART: Aminosäure
(C) STRANGFORM: Einzelstrang
(D) TOPOLOGIE: linear

(ii) ART DES MOLEKULS: Peptid
(ix) MERKMAL:

(A) NAME/SCHLÜSSEL: Peptide
(B) LAGE:12
(D) SONSTIGE ANGABEN:/product= "Xaa bedeutet Nle"

(xi) SEQUENZBESCHREIBUNG: SEQ ID NO: 75:

Asp Gly Thr Phe Thr Ser Asp Leu Ser Lys Gln Xaa Glu Glu Glu Ala

Val Arg Leu Phe Ile Glu Trp Leu Lys Asn Gly Arg

(2) ANGABEN ZU SEQ ID NO: 76:

(i) SEQUENZKENNZEICHEN:

(A) LÄNGE: 27 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 76:

```
His Ser Asp Gly Thr Phe Thr Ser Asp Leu Ser Lys Gln Xaa Glu Glu
1               5                   10                  15

Glu Ala Val Arg Leu Phe Ile Glu Trp Leu Lys
            20                  25
```

(2) ANGABEN ZU SEQ ID NO: 77:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(xi) SEQUENZBESCHREIBUNG: SEQ ID NO: 77:

```
His Lys Pro Gly Thr Phe Thr Ser Asp Leu Ser Lys Gln Ala Glu Glu
1               5                   10                  15

Glu Ala Val Arg Leu Phe Ile Glu Trp Leu Lys Asn Gly Arg
            20                  25                  30
```

(2) ANGABEN ZU SEQ ID NO: 78:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLUSSEL: Peptide
(B) LAGE:14
(D) SONSTIGE ANGABEN:/product= "Xaa bedeutet Nle"

(xi) SEQUENZBESCHREIBUNG: SEQ ID NO: 78:

```
His Ala Asp Gly Thr Phe Thr Ser Asp Leu Ser Lys Gln Xaa Glu Glu
1               5                   10                  15
```

```
Glu Ala Val Arg Leu Phe Ile Glu Trp Leu Lys Asn Gly Arg
        20                  25                  30
```

(2) ANGABEN ZU SEQ ID NO: 79:

    (i) SEQUENZKENNZEICHEN:

        (A) LÄNGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (ix) MERKMAL:

        (A) NAME/SCHLOSSEL: Peptide
        (B) LAGE:14
        (D) SONSTIGE ANGABEN:/product= "Xaa bedeutet Nle"

    (xi) SEQUENZBESCHREIBUNG: SEQ ID NO: 79:

```
His Cys Asp Gly Thr Phe Thr Ser Asp Leu Ser Lys Gln Xaa Glu Glu
1               5                   10                  15
```

```
Glu Ala Val Arg Leu Phe Ile Glu Trp Leu Lys Asn Gly Arg
        20                  25                  30
```

(2) ANGABEN ZU SEQ ID NO: 80:

    (i) SEQUENZKENNZEICHEN:

        (A) LÄNGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (ix) MERKMAL:

        (A) NAME/SCHLÜSSEL: Peptide
        (B) LAGE:14
        (D) SONSTIGE ANGABEN:/product= "Xaa bedeutet Nle"

(xi) SEQUENZBESCHREIBUNG: SEQ ID NO: 80:

His Asp Asp Gly Thr Phe Thr Ser Asp Leu Ser Lys Gln Xaa Glu Glu
1           5           10           15

Glu Ala Val Arg Leu Phe Ile Glu Trp Leu Lys Asn Gly Arg
20           25           30

(2) ANGABEN ZU SEQ ID NO: 81:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLUSSEL: Peptide
(B) LAGE:14
(D) SONSTIGE ANGABEN:/product= "Xaa bedeutet Nle"

(xi) SEQUENZBESCHREIBUNG: SEQ ID NO: 81:

His Glu Asp Gly Thr Phe Thr Ser Asp Leu Ser Lys Gln Xaa Glu Glu
1           5           10           15

Glu Ala Val Arg Leu Phe Ile Glu Trp Leu Lys Asn Gly Arg
20           25           30

(2) ANGABEN ZU SEQ ID NO: 82:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:14

(D) SONSTIGE ANGABEN:/product= "Xaa bedeutet Nle"

(xi) SEQUENZBESCHREIBUNG: SEQ ID NO: 82:

His Phe Asp Gly Thr Phe Thr Ser Asp Leu Ser Lys Gln Xaa Glu Glu

Glu Ala Val Arg Leu Phe Ile Glu Trp Leu Lys Asn Gly Arg

(2) ANGABEN ZU SEQ ID NO: 83:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 83:

His Gly Asp Gly Thr Phe Thr Ser Asp Leu Ser Lys Gln Xaa Glu Glu

Glu Ala Val Arg Leu Phe Ile Glu Trp Leu Lys Asn Gly Arg

(2) ANGABEN ZU SEQ ID NO: 84:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid

(ix) MERKMAL:

    (A) NAME/SCHLÜSSEL: Peptide
    (B) LAGE:14
    (D) SONSTIGE ANGABEN:/product= "Xaa bedeutet Nle"

(xi) SEQUENZBESCHREIBUNG: SEQ ID NO: 84:

His His Asp Gly Thr Phe Thr Ser Asp Leu Ser Lys Gln Xaa Glu Glu

1            5            10            15

Glu Ala Val Arg Leu Phe Ile Glu Trp Leu Lys Asn Gly Arg

        20            25            30

(2) ANGABEN ZU SEQ ID NO: 85:

    (i) SEQUENZKENNZEICHEN:

        (A) LANGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (ix) MERKMAL:

        (A) NAME/SCHLÜSSEL: Peptide
        (B) LAGE:14
        (D) SONSTIGE ANGABEN:/product= "Xaa bedeutet Nle"

    (xi) SEQUENZBESCHREIBUNG: SEQ ID NO: 85:

His Ile Asp Gly Thr Phe Thr Ser Asp Leu Ser Lys Gln Xaa Glu Glu

1            5            10            15

Glu Ala Val Arg Leu Phe Ile Glu Trp Leu Lys Asn Gly Arg

        20            25            30

(2) ANGABEN ZU SEQ ID NO: 86:

    (i) SEQUENZKENNZEICHEN:

        (A) LÄNGE: 30 Aminosäuren
        (B) ART: Aminosäure
        (C) STRANGFORM: Einzelstrang

(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 86:

```
His Lys Asp Gly Thr Phe Thr Ser Asp Leu Ser Lys Gln Xaa Glu Glu
1               5                   10                  15

Glu Ala Val Arg Leu Phe Ile Glu Trp Leu Lys Asn Gly Arg
        20                  25                  30
```

(2) ANGABEN ZU SEQ ID NO: 87:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 87:

```
His Leu Asp Gly Thr Phe Thr Ser Asp Leu Ser Lys Gln Xaa Glu Glu
1               5                   10                  15

Glu Ala Val Arg Leu Phe Ile Glu Trp Leu Lys Asn Gly Arg
        20                  25                  30
```

(2) ANGABEN ZU SEQ ID NO: 88:

(i) SEQUENZKENNZEICHEN:

(A) LÄNGE: 30 Aminosäuren

(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 88:

```
His Met Asp Gly Thr Phe Thr Ser Asp Leu Ser Lys Gln Xaa Glu Glu
1               5                   10                  15


Glu Ala Val Arg Leu Phe Ile Glu Trp Leu Lys Asn Gly Arg
          20                  25                  30
```

(2) ANGABEN ZU SEQ ID NO: 89:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 89:

```
His Asn Asp Gly Thr Phe Thr Ser Asp Leu Ser Lys Gln Xaa Glu Glu
1               5                   10                  15


Glu Ala Val Arg Leu Phe Ile Glu Trp Leu Lys Asn Gly Arg
          20                  25                  30
```

(2) ANGABEN ZU SEQ ID NO: 90:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 90:

His Pro Asp Gly Thr Phe Thr Ser Asp Leu Ser Lys Gln Xaa Glu Glu
1               5                   10                  15

Glu Ala Val Arg Leu Phe Ile Glu Trp Leu Lys Asn Gly Arg
        20                  25                  30

(2) ANGABEN ZU SEQ ID NO: 91:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 91:

His Gln Asp Gly Thr Phe Thr Ser Asp Leu Ser Lys Gln Xaa Glu Glu
1               5                   10                  15

Glu Ala Val Arg Leu Phe Ile Glu Trp Leu Lys Asn Gly Arg

2) ANGABEN ZU SEQ ID NO: 92:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 92:

```
His Arg Asp Gly Thr Phe Thr Ser Asp Leu Ser Lys Gln Xaa Glu Glu
1               5                   10                  15

Glu Ala Val Arg Leu Phe Ile Glu Trp Leu Lys Asn Gly Arg
            20                  25                  30
```

(2) ANGABEN ZU SEQ ID NO: 93:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 93:

```
His Thr Asp Gly Thr Phe Thr Ser Asp Leu Ser Lys Gln Xaa Glu Glu
1               5                   10                  15
```

```
Glu Ala Val Arg Leu Phe Ile Glu Trp Leu Lys Asn Gly Arg
        20                  25                  30
```

(2) ANGABEN ZU SEQ ID NO: 94:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLÜSSEL: Peptide
(B) LAGE:14
(D) SONSTIGE ANGABEN:/product= "Xaa bedeutet Nle"

(xi) SEQUENZBESCHREIBUNG: SEQ ID NO: 94:

```
His Val Asp Gly Thr Phe Thr Ser Asp Leu Ser Lys Gln Xaa Glu Glu
1               5                   10                  15
```

```
Glu Ala Val Arg Leu Phe Ile Glu Trp Leu Lys Asn Gly Arg
        20                  25                  30
```

(2) ANGABEN ZU SEQ ID NO: 95:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLUSSEL: Peptide
(B) LAGE:14
(D) SONSTIGE ANGABEN:/product= "Xaa bedeutet Nle"

(xi) SEQUENZBESCHREIBUNG: SEQ ID NO: 95:

```
His Trp Asp Gly Thr Phe Thr Ser Asp Leu Ser Lys Gln Xaa Glu Glu
1               5                   10                  15

Glu Ala Val Arg Leu Phe Ile Glu Trp Leu Lys Asn Gly Arg
        20                  25                  30
```

(2) ANGABEN ZU SEQ ID NO: 96:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(ix) MERKMAL:

(A) NAME/SCHLOSSEL: Peptide
(B) LAGE:14
(D) SONSTIGE ANGABEN:/product= "Xaa bedeutet Nle"

(xi) SEQUENZBESCHREIBUNG: SEQ ID NO: 96:

```
His Tyr Asp Gly Thr Phe Thr Ser Asp Leu Ser Lys Gln Xaa Glu Glu
1               5                   10                  15

Glu Ala Val Arg Leu Phe Ile Glu Trp Leu Lys Asn Gly Arg
        20                  25                  30
```

(2) ANGABEN ZU SEQ ID NO: 97:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(xi) SEQUENZBESCHREIBUNG: SEQ ID NO: 97:

His Ser Ala Gly Thr Phe Thr Ser Asp Leu Ser Lys Gln Ala Glu Glu
1            5           10           15

Glu Ala Val Arg Leu Phe Ile Glu Trp Leu Lys Asn Gly Arg
     20           25           30

.

(2) ANGABEN ZU SEQ ID NO: 98:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(xi) SEQUENZBESCHREIBUNG: SEQ ID NO: 98:

His Ser Asp Gly Ala Phe Thr Ser Asp Leu Ser Lys Gln Ala Glu Glu
1            5           10           15

Glu Ala Val Arg Leu Phe Ile Glu Trp Leu Lys Asn Gly Arg
     20           25           30

(2) ANGABEN ZU SEQ ID NO: 99:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(xi) SEQUENZBESCHREIBUNG: SEQ ID NO: 99:

```
His Ser Asp Gly Tyr Phe Thr Ser Asp Leu Ser Lys Gln Ala Glu Glu
1               5               10                      15
```

```
Glu Ala Val Arg Leu Phe Ile Glu Trp Leu Lys Asn Gly Arg
            20              25                      30
```

(2) ANGABEN ZU SEQ ID NO: 100:

    (i) SEQUENZKENNZEICHEN:

        (A) LÄNGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (xi) SEQUENZBESCHREIBUNG: SEQ ID NO: 100:

```
His Ser Asp Gly Thr Tyr Thr Ser Asp Leu Ser Lys Gln Ala Glu Glu
1               5               10                      15
```

```
Glu Ala Val Arg Leu Phe Ile Glu Trp Leu Lys Asn Gly Arg
            20              25                      30
```

(2) ANGABEN ZU SEQ ID NO: 101:

    (i) SEQUENZKENNZEICHEN:

        (A) LANGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (xi) SEQUENZBESCHREIBUNG: SEQ ID NO: 101:

```
His Ser Asp Gly Thr Ile Thr Ser Asp Leu Ser Lys Gln Ala Glu Glu
1               5               10              15
```

```
Glu Ala Val Arg Leu Phe Ile Glu Trp Leu Lys Asn Gly Arg
        20              25              30
```

(2) ANGABEN ZU SEQ ID NO: 102:

    (i) SEQUENZKENNZEICHEN:

        (A) LÄNGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (xi) SEQUENZBESCHREIBUNG: SEQ ID NO: 102:

```
His Ser Asp Gly Thr Phe Ser Ser Asp Leu Ser Lys Gln Ala Glu Glu
1               5               10              15
```

```
Glu Ala Val Arg Leu Phe Ile Glu Trp Leu Lys Asn Gly Arg
        20              25              30
```

(2) ANGABEN ZU SEQ ID NO: 103:

    (i) SEQUENZKENNZEICHEN:

        (A) LÄNGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (xi) SEQUENZBESCHREIBUNG: SEQ ID NO: 103:

```
His Ser Asp Gly Thr Phe Tyr Ser Asp Leu Ser Lys Gln Ala Glu Glu
1               5               10              15
```

```
Glu Ala Val Arg Leu Phe Ile Glu Trp Leu Lys Asn Gly Arg
            20              25              30
```

(2) ANGABEN ZU SEQ ID NO: 104:

    (i) SEQUENZKENNZEICHEN:

        (A) LÄNGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (xi) SEQUENZBESCHREIBUNG: SEQ ID NO: 104:

```
His Ser Asp Gly Thr Phe Thr Thr Asp Leu Ser Lys Gln Ala Glu Glu
1               5               10              15
```

```
Glu Ala Val Arg Leu Phe Ile Glu Trp Leu Lys Asn Gly Arg
            20              25              30
```

(2) ANGABEN ZU SEQ ID NO: 105:

    (i) SEQUENZKENNZEICHEN:

        (A) LÄNGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (xi) SEQUENZBESCHREIBUNG: SEQ ID NO: 105:

```
His Ser Asp Gly Thr Phe Thr Tyr Asp Leu Ser Lys Gln Ala Glu Glu
 1               5               10                  15
```

```
Glu Ala Val Arg Leu Phe Ile Glu Trp Leu Lys Asn Gly Arg
         20              25              30
```

(2) ANGABEN ZU SEQ ID NO: 106:

    (i) SEQUENZKENNZEICHEN:

      (A) LÄNGE: 30 Aminosäuren
      (B) ART: Aminosäure
      (C) STRANGFORM: Einzelstrang
      (D) TOPOLOGIE: linear

    (ii) ART DES MOLEKÜLS: Peptid
    (xi) SEQUENZBESCHREIBUNG: SEQ ID NO: 106:

```
His Ser Asp Gly Thr Phe Thr Ser Glu Leu Ser Lys Gln Ala Glu Glu
 1               5               10                  15
```

```
Glu Ala Val Arg Leu Phe Ile Glu Trp Leu Lys Asn Gly Arg
         20              25              30
```

(2) ANGABEN ZU SEQ ID NO: 107:

    (i) SEQUENZKENNZEICHEN:

      (A) LANGE: 30 Aminosäuren
      (B) ART: Aminosäure
      (C) STRANGFORM: Einzelstrang
      (D) TOPOLOGIE: linear

    (ii) ART DES MOLEKULS: Peptid
    (xi) SEQUENZBESCHREIBUNG: SEQ ID NO: 107:

His Ser Asp Gly Thr Phe Thr Ser Asp Ala Ser Lys Gln Ala Glu Glu
1                   5                   10                  15

Glu Ala Val Arg Leu Phe Ile Glu Trp Leu Lys Asn Gly Arg
                20                  25                  30

(2) ANGABEN ZU SEQ ID NO: 108:

    (i) SEQUENZKENNZEICHEN:

        (A) LÄNGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (xi) SEQUENZBESCHREIBUNG: SEQ ID NO: 108:

His Ser Asp Gly Thr Phe Thr Ser Asp Leu Ala Lys Gln Ala Glu Glu
1                   5                   10                  15

Glu Ala Val Arg Leu Phe Ile Glu Trp Leu Lys Asn Gly Arg
                20                  25                  30

(2) ANGABEN ZU SEQ ID NO: 109:

    (i) SEQUENZKENNZEICHEN:

        (A) LÄNGE: 30 Aminosäuren
        (B) ART: Aminosäure
        (C) STRANGFORM: Einzelstrang
        (D) TOPOLOGIE: linear

    (ii) ART DES MOLEKULS: Peptid
    (xi) SEQUENZBESCHREIBUNG: SEQ ID NO: 109:

```
His Gly Glu Gly Thr Phe Thr Ser Asp Leu Ser Ala Gln Ala Glu Glu
 1               5               10                  15

Glu Ala Val Arg Leu Phe Ile Glu Trp Leu Lys Asn Gly Arg
        20                 25                  30
```

(2) ANGABEN ZU SEQ ID NO: 110:

   (i) SEQUENZKENNZEICHEN:

     (A) LÄNGE: 30 Aminosäuren
     (B) ART: Aminosäure
     (C) STRANGFORM: Einzelstrang
     (D) TOPOLOGIE: linear

   (ii) ART DES MOLEKÜLS: Peptid
   (xi) SEQUENZBESCHREIBUNG: SEQ ID NO: 110:

```
His Ser Asp Gly Thr Phe Thr Ser Asp Leu Ser Lys Ala Ala Glu Glu
 1               5               10                  15

Glu Ala Val Arg Leu Phe Ile Glu Trp Leu Lys Asn Gly Arg
        20                 25                  30
```

(2) ANGABEN ZU SEQ ID NO: 111:

   (i) SEQUENZKENNZEICHEN:

     (A) LANGE: 30 Aminosäuren
     (B) ART: Aminosäure
     (C) STRANGFORM: Einzelstrang
     (D) TOPOLOGIE: linear

   (ii) ART DES MOLEKÜLS: Peptid
   (xi) SEQUENZBESCHREIBUNG: SEQ ID NO: 111:

```
His Ser Asp Gly Thr Phe Thr Ser Asp Leu Ser Lys Gln Ala Ala Glu
1               5               10              15
```

```
Glu Ala Val Arg Leu Phe Ile Glu Trp Leu Lys Asn Gly Arg
        20              25              30
```

(2) ANGABEN ZU SEQ ID NO: 112:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(xi) SEQUENZBESCHREIBUNG: SEQ ID NO: 112:

```
Lys Ser Asp Gly Thr Phe Thr Ser Asp Leu Ser Lys Gln Ala Glu Glu
1               5               10              15
```

```
Ala Ala Val Arg Leu Phe Ile Glu Trp Leu Lys Asn Gly Arg
        20              25              30
```

(2) ANGABEN ZU SEQ ID NO: 113:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(xi) SEQUENZBESCHREIBUNG: SEQ ID NO: 113:

```
His Ser Asp Gly Thr Phe Thr Ser Asp Leu Ser Lys Gln Ala Glu Glu
1                   5                   10                  15
```

```
Glu Leu Val Arg Leu Phe Ile Glu Trp Leu Lys Asn Gly Arg
            20                  25                  30
```

(2) ANGABEN ZU SEQ ID NO: 114:

    (i) SEQUENZKENNZEICHEN:

        (A) LANGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (xi) SEQUENZBESCHREIBUNG: SEQ ID NO: 114:

```
His Ser Asp Gly Thr Phe Thr Ser Asp Leu Ser Lys Gln Ala Glu Glu
1                   5                   10                  15
```

```
Glu Ala Ile Arg Leu Phe Ile Glu Trp Leu Lys Asn Gly Arg
            20                  25                  30
```

(2) ANGABEN ZU SEQ ID NO: 115:

    (i) SEQUENZKENNZEICHEN:

        (A) LÄNGE: 30 Aminosäuren
        (B) ART: Aminosäure
        (C) STRANGFORM: Einzelstrang
        (D) TOPOLOGIE: linear

    (ii) ART DES MOLEKÜLS: Peptid
    (xi) SEQUENZBESCHREIBUNG: SEQ ID NO: 115:

His Ser Asp Gly Thr Phe Thr Ser Asp Leu Ser Lys Gln Ala Glu Glu

Glu Ala Val Ala Leu Phe Ile Glu Trp Leu Lys Asn Gly Arg

(2) ANGABEN ZU SEQ ID NO: 116:

(i) SEQUENZKENNZEICHEN:

(A) LÄNGE: 30 Aminosäuren
(B) ART: Aminosäure
(C) STRANGFORM: Einzelstrang
(D) TOPOLOGIE: linear

(ii) ART DES MOLEKÜLS: Peptid
(xi) SEQUENZBESCHREIBUNG: SEQ ID NO: 116:

His Ser Asp Gly Thr Phe Thr Ser Asp Leu Ser Lys Gln Ala Glu Glu

Glu Ala Val Arg Leu Tyr Ile Glu Trp Leu Lys Asn Gly Arg

(2) ANGABEN ZU SEQ ID NO: 117:

(i) SEQUENZKENNZEICHEN:

(A) LANGE: 30 Aminosäuren
(B) ART: Aminosäure
(C) STRANGFORM: Einzelstrang
(D) TOPOLOGIE: linear

(ii) ART DES MOLEKÜLS: Peptid
(xi) SEQUENZBESCHREIBUNG: SEQ ID NO: 117:

His Ser Asp Gly Thr Phe Thr Ser Asp Leu Ser Lys Gln Ala Glu Glu

```
Glu Ala Val Arg Leu Phe Val Glu Trp Leu Lys Asn Gly Arg
            20                  25                  30
```

(2) ANGABEN ZU SEQ ID NO: 118:

   (i) SEQUENZKENNZEICHEN:

      (A) LANGE: 30 Aminosäuren
      (B) ART: Aminosäure
      (C) STRANGFORM: Einzelstrang
      (D) TOPOLOGIE: linear

   (ii) ART DES MOLEKÜLS: Peptid
   (xi) SEQUENZBESCHREIBUNG: SEQ ID NO: 118:

```
His Ser Asp Gly Thr Phe Thr Ser Asp Leu Ser Lys Gln Ala Glu Glu
1               5                   10                  15

Glu Ala Val Arg Leu Phe Ile Leu Trp Leu Lys Asn Gly Arg
            20                  25                  30
```

**Patentansprüche**

**1.**   Peptid, **dadurch gekennzeichnet, dass** es die Sequenz 1 oder 2 aufweist

```
SEQ ID NO:1

1           5               10              15
H   S   D   G   T   F   T   S   D   L   S   K   Q   M   E   E   E   A   V
20              25              30
R   L   F   I   E   W   L   K   N   G   X
```

**SEQ ID NO: 2**

```
1             5                10                15
H   G   E   G   T   F   T   S   D   L   S   K   Q   M   E   E   A   V
20               25  -           30
R   L   F   I   E   W   L   K   N   G   X
```

wobei X eine proteinogene oder nicht-proteinogene Aminosäure ausser Glycin bedeutet,
die Aminosäuren in Position 1, 2, 28, 29 oder 30 unabhängig vaneinander einzeln oder zusammen Teil der Sequenz sein können und der
N-Terminus durch $NR_1R_2$ dargestellt wird, wobei

$R_1$ Wasserstoff, Acetyl, Trifluoracetyl, Adamantyl, Fmoc, Z, Boc, Alloc, $C_1$-$C_6$-Alkyl, $C_2$-$C_8$ Alkenyl oder $C_7$-$C_9$Aralkyl,
$R_2$ Wasserstoff, Acetyl, Trifluoracetyl, Adamantyl, Fmoc, Z, Boc, Alloc, $C_4$-$C_6$-Alkyl, $C_2$-$C_8$ Alkenyl oder $C_7$-$C_9$ Aralkyl, bedeuten

und der C-Teminus durch $COR_3$ dargestellt wird, wobei

$R_3$ gleich $OR_4$ oder $NR_4R_5$
mit $R_4$ gleich Wasserstoff oder $C_1$-$C_6$-Alkyl
mit $R_5$ gleich Wasserstoff oder $C_1C_6$-Alkyl

bedeutet, sowie deren physilogisch verträglichen Salze und Ester.

2. Peptide nach Anspruch 1, **dadurch gekennzeichnet, dass** mindestens eine aber höchstens 11 der folgenden Modifikationen an der Aminosäurekette erfolgt sind:

(a) Die α-Aminosäure in Position 1 ist Ala, Gly oder Lys; oder
(b) Die α-Aminosäure in Position 2 ist Ser, Thr, Gly, Ala, Val, Ile oder Leu; oder
(c) Die α-Aminosäure in Position 3 ist Glu, Asp or Ala; oder
(d) Die Aminosäure in Position 4 ist Ala; oder
(e) Die α-Aminosäure in Position 5 ist Ser, Tyr oder Ala; oder
(f) Die α-Aminosäure in Position 6 ist Ala, Ile, Val, Leu oder Tyr; oder
(g) Die α-Aminosäure in Position 7 ist Ala, Tyr oder Ser; oder
(h) Die α-Aminosäure in Position 8 ist Ala, Tyr oder Thr; oder
(i) Die α-Aminosäure in Position 9 ist Ala oder Glu; oder
(j) Die Aminosäuren in Position 10, 11, 12, 15, 16, 17, 18, 19, 20, 21, 24, 28, 29 sind unabhängig voneinander eine proteinogene L-Aminosäure; oder
(k) Die α-Aminosäure in Position 13 ist eine neutrale proteinogene L-Aminosäure; oder
(l) Die α-Aminosäure in Position 14 wird ersetzt durch Nle, D-Nle, Ala, D-Ala, Ile, D-Ile, Val oder D-Val; oder
(m) Die α-Aminosäure in Position 22 ist Tyr, Leu oder Val; oder
(n) Die α-Aminosäure in Position 23 ist Leu, Val, Tyr oder Phe; oder
(o) Die α-Aminosäure in Position 25, 26 or 27 ist eine neutrale, proteinogene L-Aminosäure; oder
(p) Die α-Aminosäure in Position 30 ist Arg oder Tyr.

3. Peptid nach einem der Ansprüche 1 oder 2, **dadurch gekennzeichnet, dass** es nur proteinogene Aminosäuren enthält.

4. Peptid nach einem der Ansprüche 1-3, **dadurch gekennzeichnet, dass** in Position 2 gegenüber der Sequenz 1 oder 2 ein Aminosäureaustausch erfolgt ist.

5. Peptid nach einem der Ansprüche 1-4, **dadurch gekennzeichnet, dass** in Position 14 gegenüber der Sequenz 1 oder 2 ein Aminosäureaustausch erfolgt ist.

6.  Peptid nach einem der Ansprüche 1-5, **dadurch gekennzeichnet, dass** in Position 3 gegenüber der Sequenz 1 oder 2 ein Aminosäureaustausch erfolgt ist.

7.  Peptid nach einem der Ansprüche 1-6, **dadurch gekennzeichnet, dass** es eine der Sequenzen 3 bis 120 aufweist.

8.  Peptid nach einem der Ansprüche 1-6, **dadurch gekennzeichnet, dass** es eine der Sequenzen 5, 68, 69, 71, 78-82 oder 84-91 aufweist, wobei der N-Terminus durch $NR_1R_2$ dargestellt wird, wobei

> $R_1$ Wasserstoff, Acetyl, Trifluoracetyl, Adamantyl, Fmoc, Z, Boc, Alloc, $C_1$-$C_6$-Alkyl, $C_2$-$C_8$ Alkenyl oder $C_7$-$C_9$Aralkyl,
> $R_2$ Wasserstoff, Acetyl, Trifluoracetyl, Adamantyl, Fmoc, Z, Boc, Alloc, $C_4$-$C_6$-Alkyl, $C_2$-$C_8$ Alkenyl oder $C_7$-$C_9$Aralkyl, bedeuten

und der C-Teminus durch $COR_3$ dargestellt wird, wobei

> $R_3$ gleich $OR_4$ oder $NR_4R_5$
> mit $R_4$ gleich Wasserstoff oder $C_1$-$C_6$-Alkyl
> mit $R_5$ gleich Wasserstoff oder $C_1$-$C_6$-Alkyl

bedeutet, sowie deren physilogisch verträglichen Salze und Ester.

9.  Peptid nach einem der Ansprüche 1-8, **dadurch gekennzeichnet, dass** es die Insulinfreisetzung stimuliert.

10. Arzneimittel enthaltend neben üblichen Trägern und Hilfsstoffen mindestens ein Peptid nach einem der Ansprüche 1-8.

11. Verwendung von Peptiden nach einem der Ansprüche 1-8 zur Herstellung von pharmazeutischen Zusammensetzungen zur Behandlung von Diabetes.

**Revendications**

1.  Peptide **caractérisé en ce qu'**il présente la séquence 1 ou la séquence 2

```
SEQ ID N° 1
1          5            10            15
H  S  D  G  T  F  T  S  D  L  S  K  Q  M  E  E  A  V
20            25            30
R  L  F  I  E  W  L  K  N  G  X


SEQ ID N° 2
1          5            10            15
H  G  E  G  T  F  T  S  D  L  S  K  Q  M  E  E  A  V
20            25 -          30
R  L  F  I  E  W  L  K  N  G  X
```

dans laquelle X désigne un acide aminé protéinogène ou non protéinogène, excepté la glycine, les acides aminés dans la position 1, 2, 28, 29 ou 30, indépendamment les uns des autres ou conjointement, peuvent faire partie de la séquence et l'extrémité terminale N est représentée par un groupe $NR_1R_2$, dans lequel

> $R_1$ représente l'hydrogène, un groupe acétyle, trifluoracétyle, adamantyle, Fmoc, Z, Boc, Alloc, alkyle en $C_1$ à

$C_6$, alcényle en $C_2$ à $C_8$ ou aralkyle en $C_7$ à $C_9$,
$R_2$ représente l'hydrogène, un groupe acétyle, trifluoracétyle, adamantyle, Fmoc, Z, Boc, Alloc, alkyle en $C_4$ à $C_6$, alcényle en $C_2$ à $C_8$ ou aralkyle en $C_7$ à $C_9$,

et l'extrémité terminale C est représentée par un groupe $COR_3$, dans lequel

$R_3$ représente $OR_4$ ou $NR_4R_5$
où $R_4$ est l'hydrogène ou un reste alkyle en $C_1$ à $C_6$
$R_5$ est l'hydrogène ou un reste alkyle en $C_1$ à $C_6$,

ainsi que leurs sels et leurs esters acceptables du point de vue physiologique.

**2.** Peptides suivant la revendication 1, **caractérisés en ce qu'**au moins l'une mais au maximum 11 des modifications suivantes sont produites sur la chaîne des aminoacides :

(a) L'α-aminoacide en position 1 est Ala, Gly ou Lys ; ou bien
(b) L'α-aminoacide en position 2 est Ser, Thr, Gly, Ala, Val, Ile ou Leu ; ou bien
(c) L'α-aminoacide en position 3 est Glu, Asp ou Ala ; ou bien
(d) L'aminoacide en position 4 est Ala ; ou bien
(e) L'α-aminoacide en position 5 est Ser, Tyr ou Ala ; ou bien
(f) L'α-aminoacide en position 6 est Ala, Ile, Val, Leu ou Tyr ; ou bien
(g) L'α-aminoacide en position 7 est Ala, Tyr ou Ser ; ou bien
(h) L'α-aminoacide en position 8 est Ala, Tyr ou Thr ; ou bien
(i) L'α-aminoacide en position 9 est Ala ou Glu *;* ou bien
(j) Les aminoacides en positions 10, 11, 12, 15, 16, 17, 18, 19, 20, 21, 24, 28, 29 sont, indépendamment les uns des autres, un L-aminoacide protéinogène ; ou bien
(k) L'α-aminoacide en position 13 est un L-aminoacide protéinogène neutre ; ou bien
(l) L'α-aminoacide en position 14 est remplacé par Nle, D-Nle, Ala, D-Ala, Ile, D-Ile, Val ou D-Val ; ou bien
(m) L'α-aminoacide en position 22 est Tyr, Leu ou Val ; ou bien
(n) L'α-aminoacide en position 23 est Leu, Val, Tyr ou Phe ; ou bien
(o) L'α-aminoacide en position 25, 26 ou 27 est un L-aminoacide protéinogène neutre ; ou bien
(p) L'α-aminoacide en position 30 est Arg ou Tyr.

**3.** Peptide suivant l'une des revendications 1 ou 2, **caractérisé en ce qu'**il ne contient que des aminoacides protéinogènes.

**4.** Peptide suivant l'une des revendications 1 à 3, **caractérisé en ce qu'**un échange d'aminoacides est effectué en position 2 par rapport à la séquence 1 ou 2.

**5.** Peptide suivant l'une des revendications 1 à 4, **caractérisé en ce qu'**un échange d'aminoacides est effectué en position 14 par rapport à la séquence 1 ou 2.

**6.** Peptide suivant l'une des revendications 1 à 5, **caractérisé en ce qu'**un échange d'aminoacides est effectué en position 3 par rapport à la séquence 1 ou 2.

**7.** Peptide suivant l'une des revendications 1 à 6, **caractérisé en ce qu'**il présente l'une des séquences 3 à 120.

**8.** Peptide suivant l'une des revendications 1 à 6, **caractérisé en ce qu'**il présente l'une des séquences 5, 68, 69, 71, 78-82 ou 84-91, l'extrémité terminale N étant représentée par un groupe $NR_1R_2$, dans lequel

$R_1$ représente l'hydrogène, un reste acétyle, trifluoracétyle, adamantyle, Fmoc, Z, Boc, Alloc, alkyle en $C_1$ à $C_6$, alcényle en $C_2$ à $C_8$ ou aralkyle en $C_7$ à $C_9$,
$R_2$ représente l'hydrogène, un reste acétyle, trifluoracétyle, adamantyle, Fmoc, Z, Boc, Alloc, alkyle en $C_4$ à $C_6$, alcényle en $C_2$ à $C_8$ ou aralkyle en $C_7$ à $C_9$,

et l'extrémité terminale C est représentée par un groupe $COR_3$, dans lequel

$R_3$ représente $OR_4$ ou $NR_4R_5$

où $R_4$ représente l'hydrogène ou un reste alkyle en $C_1$ à $C_6$
$R_5$ représente l'hydrogène ou un reste alkyle en $C_1$ à $C_6$,

ainsi que leurs sels et esters acceptables du point de vue physiologique.

**9.** Peptide suivant l'une des revendications 1 à 8, **caractérisé en ce qu'**il stimule la libération d'insuline.

**10.** Médicament contenant, à côté de supports et de substances auxiliaires classiques, au moins un peptide suivant l'une des revendications 1 à 8.

**11.** Utilisation de peptides suivant l'une des revendications 1 à 8 pour la préparation de compositions pharmaceutiques destinées au traitement du diabète.

**Claims**

**1.** Peptide, **characterized in that** it exhibits the sequence 1 or 2

SEQ ID NO:1

```
1           5               10              15
H   S   D   G   T   F   T   S   D   L   S   K   Q   M   E   E   E   A   V
20              25              30
R   L   F   I   E   W   L   K   N   G   X
```

SEQ ID NO: 2

```
1           5               10              15
H   G   E   G   T   F   T   S   D   L   S   K   Q   M   E   E   E   A   V
20              25  -           30
R   L   F   I   E   W   L   K   N   G   X
```

where X is a proteinogenic or nonproteinogenic amino acid apart from glycine,
the amino acids in position 1, 2, 28, 29 or 30 can, independently of each other, individually or together, be part of the sequence, and the N terminus is represented by $NR_1R_2$, where

$R_1$ is hydrogen, acetyl, trifluoroacetyl, adamantyl, Fmoc, Z, Boc, Alloc, $C_1$-$C_6$-alkyl, $C_2$-$C_8$ alkenyl or $C_7$-$C_9$ aralkyl,
$R_2$ is hydrogen, acetyl, trifluoroacetyl, adamantyl, Fmoc, Z, Boc, Alloc, $C_4$-$C_6$-alkyl, $C_2$-$C_8$ alkenyl or $C_7$-$C_9$ aralkyl,

and the C terminus is represented by $COR_3$, where

$R_3$ is $OR_4$ or $NR_4R_5$,
where $R_4$ is hydrogen or $C_1$-$C_6$-alkyl, and
where $R_5$ is hydrogen or $C_1$-$C_6$-alkyl,

and also the physiologically tolerated salts and esters thereof.

**2.** Peptides according to Claim 1, **characterized in that** at least one, but at most 11, of the following modifications to the amino acid chain have taken place:

(a) the α-amino acid in position 1 is Ala, Gly or Lys; or
(b) the α-amino acid in position 2 is Ser, Thr, Gly, Ala, Val, Ile or Leu; or
(c) the α-amino acid in position 3 is Glu, Asp or Ala; or
(d) the amino acid in position 4 is Ala; or
(e) the α-amino acid in position 5 is Ser, Tyr or Ala; or
(f) the α-amino acid in position 6 is Ala, Ile, Val, Leu or Tyr; or
(g) the α-amino acid in position 7 is Ala, Tyr or Ser; or
(h) the α-amino acid in position 8 is Ala, Tyr or Thr; or
(i) the α-amino acid in position 9 is Ala or Glu; or
(j) the amino acids in positions 10, 11, 12, 15, 16, 17, 18, 19, 20, 21, 24, 28 and 29 are, independent of each other, a proteinogenic L-amino acid; or
(k) the α-amino acid in position 13 is a neutral proteinogenic L-amino acid; or
(l) the α-amino acid in position 14 is replaced with Nle, D-Nle, Ala, D-Ala, Ile, D-Ile, Val or D-Val; or
(m) the α-amino acid in position 22 is Tyr, Leu or Val; or
(n) the α-amino acid in position 23 is Leu, Val, Tyr or Phe; or
(o) the α-amino acid in position 25, 26 or 27 is a neutral, proteinogenic L-amino acid; or
(p) the α-amino acid in position 30 is Arg or Tyr.

3. Peptide according to Claim 1 or 2, **characterized in that** it only contains proteinogenic amino acids.

4. Peptide according to one of Claims 1-3, **characterized in that** an amino acid substitution has taken place in position 2 with regard to sequence 1 or 2.

5. Peptide according to one of Claims 1-4, **characterized in that** an amino acid substitution has taken place in position 14 with regard to sequence 1 or 2.

6. Peptide according to one of Claims 1-5, **characterized in that** an amino acid substitution has taken place in position 3 with regard to sequence 1 or 2.

7. Peptide according to one of Claims 1-6, **characterized in that** it exhibits one of the sequences 3 to 120.

8. Peptide according to one of Claims 1-6, **characterized in that** it exhibits one of the sequences 5, 68, 69, 71, 78-82 or 84-91, where the N terminus is represented by $NR_1R_2$, where

$R_1$ is hydrogen, acetyl, trifluoroacetyl, adamantyl, Fmoc, Z, Boc, Alloc, $C_1$-$C_6$-alkyl, $C_2$-$C_8$ alkenyl or $C_7$-$C_9$ aralkyl,
$R_2$ is hydrogen, acetyl, trifluoroacetyl, adamantyl, Fmoc, Z, Boc, Alloc, $C_4$-$C_6$-alkyl, $C_2$-$C_8$ alkenyl or $C_7$-$C_9$ aralkyl,

and the C terminus is represented by $COR_3$, where

$R_3$ is $OR_4$ or $NR_4R_5$,
where $R_4$ is hydrogen or $C_1$-$C_6$-alkyl, and
where $R_5$ is hydrogen or $C_1$-$C_6$-alkyl,

and also the physiologically tolerated salts and esters thereof.

9. Peptide according to one of Claims 1-8, **characterized in that** it stimulates insulin release.

10. Pharmaceutical comprising, in addition to customary excipients and auxiliary substances, at least one peptide according to one of Claims 1-8.

11. Use of peptides according to one of Claims 1-8 for producing pharmaceutical compositions for treating diabetes.